# EUROPEAN PATENT SPECIFICATION

(11) **EP 1 006 845 B1**
(45) Date of publication and mention of the grant of the patent: **28.06.2006**
(21) Application number: 98911766.8
(22) Date of filing: 13.03.1998
(51) Int. Cl.: A47D 9/02, A47C 21/00, A61H 1/00

(54) **RECIPROCATING MOVEMENT PLATFORM FOR SHIFTING SUBJECT TO AND FRO IN HEADWARDS-FOOTWARDS DIRECTION**
HIN- UND HERBEWEGENDE PLATTFORM FÜR DIE BEWEGUNG EINER PERSON NACH VORNE UND HINTEN IN KOPF-FUSS RICHTUNG
PLATE-FORME A MOUVEMENT DE VA-ET-VIENT SERVANT A DEPLACER UN SUJET D'AVANT EN ARRIERE DANS UN SENS TETE-PIEDS

(30) Priority: 14.03.1997 US 40457 P; 05.11.1997 US 64541 P
(43) Date of publication of application: 14.06.2000
(73) Proprietor: NON-INVASIVE MONITORING SYSTEMS, INC., Miami Beach, FL 33139 (US)
(72) Inventor: SACKNER, Marvin, A., Miami Beach, FL 33139 (US); INMAN, D., Michael, Miami, FL 33138 (US); MEICHNER, William, J., Royal Oak, MD 21662 (US)
(74) Representative: Joly, Jean-Jacques
(86) International application number: PCT/US1998/005291
(87) International publication number: WO 1998/039996

(56) References cited:
- US-A- 3 950 799
- US-A- 4 619 270
- US-A- 4 934 997
- US-A- 5 520 614

## Description

The present invention relates to a platform assembly with a movable platform operable in an oscillatory motion so that a subject on the platform moves in a headwards and footwards direction with the platform. Currently contemplated applications of the invention will utilize variations in the speed, frequency and symmetry of oscillatory movement of the platform and include at least the following: sleep induction, apnea prevention, awakening functions, relief from restless legs syndrome and painful legs and moving toes syndrome, non-invasive motion ventilation, non-invasive vibratory ventilation, non-invasive cardiopulmonary resuscitation, non-invasive counter pulsation, augmentation of stroke volume, non-invasive cardiopulmonary bypass support, mediator release, and stimulation of bowel motility.

### 2. Description of the Related Art

It has long been recognized that many physiological characteristics such as breathing rates and heartbeats of humans and other animals are cyclic and that bodies respond to certain types of oscillatory stimulations with beneficial results. For instance, parents have long recognized that rocking a baby back and forth in a headwards-footwards direction soothes their child and hastens the onset of the infant's sleep. Many devices have been designed and marketed to accomplish this end. In addition to parental observation, scientific evidence indicates that babies have better developed proprioceptive-vestibular receptive systems than adults. This scientific evidence has been the basis for the development of devices which seek to soothe the baby in a simulated maternal development environment through such features as movements in a specially designed bed to simulate the mother's walking while the baby was a fetus, in conjunction with simulated sounding of maternal breath sounds, heart sounds and gastrointestinal sounds within such a system.

Furthermore, it appears that gentle, small oscillations on a waterbed may diminish the frequency of apneas in preterm infants. Prior art devices for preventing apnea include air mattresses with a bubble chamber, devices which induce auditory stimulation, devices which induce a vibration in a peripheral sensory area of the infant, a bed with a suspension and drive system to simulate motion experienced by a fetus while the mother is walking, and apparatus for producing mechanical vibrations which simulate the mother's heartbeat. These devices are passive in that they constantly produce their aural or mechanical stimulants. Others use non-invasive respiratory monitoring systems to trigger external stimulants to end an adverse cardiorespiratory event, such as an apneic event. Such triggered systems employ harsher rocking motions and vibrations to determine the adverse cardiorespiratory events. For example, it is recommended that the baby be stimulated by touching and gently shaking to cause awakening and thereby terminate an apneic event.

US Patent No 4,619,270 discloses a crib device configured to vigorously shake a baby lying therein from side to side or up and down (i.e. vertically) when the baby experiences respiratory or cardiac problems.

As mentioned above, the breathing rate of a respiratory system is a clinical physiological function of the body. Many forms of vibration and oscillatory stimuli which utilise the cyclical characteristics of the respiratory system are used for medical and/or experimental purposes. For instance, it is known that external chest vibrations in combination with tracheal gas insufflation, which is a form of high frequency vibration ventilation (HFVV), is an effective means of artificial ventilation in experimental animals. For example, when anesthetized paralysed dogs are placed in the lateral decubitus position with their chest on a vibrating plate at a frequency of 15-30 Hz and an amplitude of 2-4 mm upward and downward, and a low flow of air is insufflated into the trachea at the level of the carina, adequate gas exchange is maintained. It has also been found that vibration of the chest wall reduces breathlessness. Vibration of the chest wall inspiratory muscles during inspiration (in-phase) reduces breathlessness associated with hypercapnia and resistive loading in normal subjects and patients with chronic obstructive lung disease. Vibration is typically applied using two standard physiotherapy vibrators - - at a vibration amplitude of 2 mm at 120 Hz - - manually triggered from the inspiratory flow signal displayed on a storage oscilloscope. However, no devices or methods are known where vibration or any other oscillatory motion alone vllill support ventilation.

Conventional positive pressure mechanical ventilators or positive pressure high frequency oscillatory ventilators support ventilation by repeatedly introducing volumes of air into the lungs and then releasing. A problem with these conventional methods of supporting ventilation is that a rather large pressure is required to inflate the lungs. The high positive pressure inflations of the lungs created by mechanical ventilators may damage the lungs in a phenomenon designated "barotrauma". Recent studies have made it apparent that lung injuries may also result from high tidal volumes or "volutrauma", the main determinant of which is the end-inspiratory volume. A ventilator that adequately exchanges gasses in the lungs and that requires less pressure in the lungs would reduce the effects and/or the occurrences of "barotrauma" and "volutrauma". High frequency oscillation ventilation is used with reduced acute and chronic lung injury. However, conventional high-frequency oscillation ventilation requires the insertion of an intubated airway which may not be appropriate under some of the circumstances in which mechanical ventilatory support is required.

The heart beat is another cyclical physiological function of the body. Many medical procedures, such as cardiovascular resuscitation, take advantage of the cyclical nature of the heart rhythm. Although many manual and automatic methods have been utilized for cardiopulmonary resuscitation, the perfect method has yet to be described. Active mechanical compression and decompression is the most recent method to be advocated; its advantage over standard compression is that the addition of active decompression facilitates venous return as intrathoracic pressure becomes negative relative to atmospheric pressure. And recently, a CPR method using a phased chest and abdominal compression-depression with a Lifestick® resuscitator for CPR was reported; this method suggests that active compression and decompression of both the thorax and abdomen with a phase shift of 180° is optimal, and subsequent trials have suggested that further improvement could be attained with a phase shift of 240°.

Another medical procedure which utilizes the cyclical nature of the heart beat and used for improving blood flow is called external enhanced counterpulsation, the goals of which are to decrease the pressure generated by the myocardium during systole and to augment the function of the compromised myocardium by increasing coronary blood flow. External enhanced counterpulsation is performed by compressing the vascular beds within the muscles of the legs and thighs, including the buttocks, in a sequential manner progressing from the calves to the lower and then the upper thighs. This procedure is accomplished by placing and selectively inflating air inflatable bladders around the limbs. Timing of compression is controlled by an electrocardiogram, with the activation of a bladder compression in the vicinity of the electrocardiographic T wave (diastole). This causes increased blood flow and pressure to reach the coronary vessels in diastole at the lowest intramyocardial tension. Compression also increases venous return and cardiac output. The external pressure is then released during the next subsequent R wave (systole), causing systolic unloading and decreasing cardiac work. This treatment improves survival in patients with cardiogenic shock after myocardial infarction, to significantly reduce mortality during acute myocardial infarction, and to improve hemodynamics in chronic angina pectoris. However, the process is complex.

Yet another procedure that takes advantage of the cyclical nature of the heart rhythm is high frequency jet ventilation to augment cardiac stroke volume. Because both systemic venous return and factors determining left ventricular performance may vary over the cardiac cycle, phasic increases in intrathoracic pressure, as produced by high frequency jet ventilation, may differentially affect ventricular preload or afterload if delivered at specific points in the cardiac cycle. When contractility is normal, selective increases of intrathoracic pressure at end-diastole minimally impairs ventricular loading and causes mild hemodynamic deterioration. When cardiac contractility is impaired and filling pressures elevated, selectively increasing intrathoracic pressure during systole is associated with increased stroke volume despite decreases in left ventricular filling pressure. In acute mitral regurgitation, increasing intrathoracic pressure by increasing the pressure gradient for left ventricular ejection augments cardiac output. This has been demonstrated by utilization of high frequency jet ventilation triggered off either cardiac systolic or diastole in an animal model. As compared with conventional positive pressure mechanical ventilation, systolic synchronous jet ventilation induced a greater increase in stroke volume than diastolic synchronous jet ventilation. However, this procedure is also very complex and is highly invasive to the subject on which it is being performed.

Recently, a large body of evidence has accumulated indicating that increases in the amplitude and frequency of blood flow and intravascular shear stress enhance vascular function and structure. One method for increasing the cycle of blood flow and intravascular sheer stress is exercise. Increases in shear stress on the vascular endothelium caused by increases in blood flow and pulstility lead to an increased release of beneficial mediators such as nitric oxide, prostacyclin, renin, and tissue plasminogen activator. Decreased shear stress causes less release of these mediators which promotes development of arteriosclerotic lesions. It is believed that exercise-induced increases in blood flow and shear stress, by increasing the release of nitric oxide and prostacyclin, augment endothelium-dependent vasodilation and inhibit multiple processes involved in atherogenesis and restenosis. Congestive heart failure, which is marked by decreased cardiac output, results in reduced shear stress on the endothelial cells and less beneficial mediator release. As mentioned above, the best way to maintain proper levels is to exercise; however, it is difficult for some people to exercise because of either physical constraints or respiratory problems.

Another problem precipitated by rest is restless legs syndrome and painful legs and moving toes syndrome. Restless legs syndrome is a common problem that is precipitated by rest and relieved by activity. It is treated with a variety of medications, all of which have major side effects.

Finally, according to the National Digestive Diseases Information Clearing House on Constipation, lack of exercise can lead to constipation. Constipation often occurs after an accident or during an illness when one is bedridden and cannot exercise.

### SUMMARY OF THE INVENTION

It is an object of the present invention to provide a reciprocating movement platform that is operable for rocking a subject in a headwards and footwards direction to induce the onset of sleep, to prevent or minimize apneas, to rapidly shake the subject in the headwards and footwards directions for waking the subject if the subject is experiencing an adverse cardiorespiratory event, to relieve the effects of restless legs syndrome and painful legs and moving toes syndrome, to oscillate the subject in the headwards and footwards directions for non-invasive motion ventilation, non-invasive vibratory ventilation, non-invasive cardiopulmonary resuscitation, non-invasive counter pulsation, augmentation of stroke volume and non-invasive cardiopulmonary bypass support, to produce mediator release from endothelial linings of a vascular system, and to stimulate bowel motility.

The platform comprises a board formed of a stiff material on which a mattress or other type of cushioning object may be placed. The platform is movably connected to a frame using displacement modules that operatively move the platform in a headwards and footwards direction. The displacement modules may comprise electric or pneumatic solenoids. The modules may also comprise an AC or DC motor with a linear shaft or a hydraulic actuator. The movement characteristics of the platform are controlled by the displacement modules with control signals originating from a processor to control the amplitude, frequency and acceleration of movement. The processor further comprises a memory in which may be stored motion templates which include information relating to specific types of motions. For instance, a specific oscillatory motion for inducing sleep based on the rocking motion of carriages and/or patting a baby to sleep can be stored in the memory. The platform may also be gently rocked to prevent apneic events. These motions of the platform may be manually adjusted to suit individual subjects. The adjusted motions may be recorded using a detector such as an accelerometer, or any other type of detector for indicating the motion characteristics of the platform such as a speedometer or a position detector, by downloading the information from the detector to the processor memory. The recorded characteristics of the motion may thereafter be accessed for recreating that particular motion at a later time. Signals from the detector may be used as a measure of quality control of the effectiveness of the platform. The detector may also be used as a safety device whereby the processor discontinues movement of the displacement modules when platform movement causes the output of the detector to exceed a predetermined limiting value. Instead of using preset templates, the motion of the platform may also be manually adjustable by dynamically varying the characteristics of the motion, such as its frequency, amplitude, and acceleration, during the reciprocal movement using an input device for determining the type of the motion.

The platform assembly optionally includes a monitoring device for triggering a specific motion of the platform upon the occurrence of an event. In a simple embodiment, the event could be the crying of a baby; in that case, the monitor may be a microphone and the processor may initiate a gentle rocking motion of the platform if a preset audio level is exceeded. The event could also be an adverse cardiorespiratory event, such as central, mixed, and obstructive apneas, prolonged apnea, pulse waveform validated arterial oxygen saturation, heart rate changes from an electrocardiogram, severe hypoxemia, and/or severe bradycardia. The processor responds to this event by rapidly shaking the platform to wake the subject and end the adverse event. If the adverse event is not terminated by the shaking, the additional stimulatory modules or functionality may be activated serially or in parallel to the shaking. The additional stimulatory modules may include a strong light source directed at the face of the subject, a voice sound calling the subject to wake up, and/or a pulsating air column directed onto the skin. Other types of stimuli directly contacting the subject may also be used. However, the preferred method for terminating this type of adverse event is to employ stimuli that do not directly contact the subject so as to avoid potential trauma caused by devices acting in direct contact with the body.

The monitoring device for monitoring these adverse events is preferably based on Respitrace® technology, including plethysmography, electrocardiography, and pulse wave validated pulse oximetry.

The reciprocating platform of the invention is also operable for oscillating at or near the resonance frequency of the respiratory system of a subject on the platform, such as in the range of 3 - 12 Hz at an amplitude of approximately 1.5 - 2.5 cm, to provide an assisted ventilation and/or as a source of ventilation for the subject. The platform is additionally operable for supporting ventilation or assisting ventilation at the frequency of normal breathing rates, and can also be vibrated at high frequencies (15-30 Hz) and low amplitudes (0.2 - 0.5 cm) to alleviate dyspnea in lung disease and as an aid in tracheal gas insufflation. Prior art devices for inducing vibrations include only small vibrating devices placed on or below the torso of the subject; the platform of the present invention, however, induces vibration throughout the entire chest area and thereby greatly enhances the effects of the vibration.

At the high frequency oscillations 4-10 Hz, the inventive platform also 1) increases synthesis in the alveoli and release of surfactant into the airways from the alveoli, 2) evenly distributes pharmacological agents within the lungs, and 3) a clears retained bronchopulmonary secretions by oscillatory two-phase gas-liquid interaction.

The oscillations of the reciprocating platform at 3-12 Hz also serves as cardiopulmonary or cardiac support for 1) cardiopulmonary resuscitation, 2) counterpulsation, 3) augmentation of cardiac stroke volume, and 4) non-invasive cardiopulmonary bypass.

Finally, the oscillatory motion of the platform may substitute for the beneficial effects of exercise by promoting endothelial shear stress that also serves as a source for endogenous nitric oxide release for treatment of entities in which this mediator is indicated. These entities include, by way of non-limiting example, septic shock, chronic heart failure, cerebrovascular accidents, and pulmonary hypertension. The motion platform may also aid in the prevention of restless legs syndrome, and aid in the prevention and treatment of constipation in bedridden patients.

The various features of novelty which characterize the invention are pointed out with particularity in the claims annexed to and forming a part of the disclosure. For a better understanding of the invention, its operating advantages, and specific objects attained by its use, reference should be had to the drawings and descriptive matter in which there are illustrated and described preferred embodiments of the invention.

### BRIEF DESCRIPTION OF THE DRAWINGφφS

In the drawings, wherein like reference characters denote similar elements throughout the several views:
Fig. 1 is a schematic diagram of an embodiment of the platform assembly of the present invention;
Fig. 1a depicts an alternate construction of the platform of the assembly of Fig. 1;
Figs. 2a, 2b, and 2c show the displacement modules and platform of the assembly of Fig. 1;
Fig. 2d depicts another embodiment of the platform of the assembly.
Fig. 3a and 3b show a subject on the platform at the operating positions of the platform shown in Figs. 2a and 2b, respectively;
Fig. 4 is a block diagram of the control circuit for the platform assembly of the present invention;
Fig. 5 is a schematic depiction of a connection of the pneumotachograph and the bias flow circuit attached to the airway of the subject through a T-piece during tests of the platform assembly; and
Figs. 6-15 are a series of graphs showing the physiologic waveforms for a third piglet during NIMCPR.

### DETAILED DESCRIPTION OF THE PRESENTLY PREFERRED EMBODIMENTS

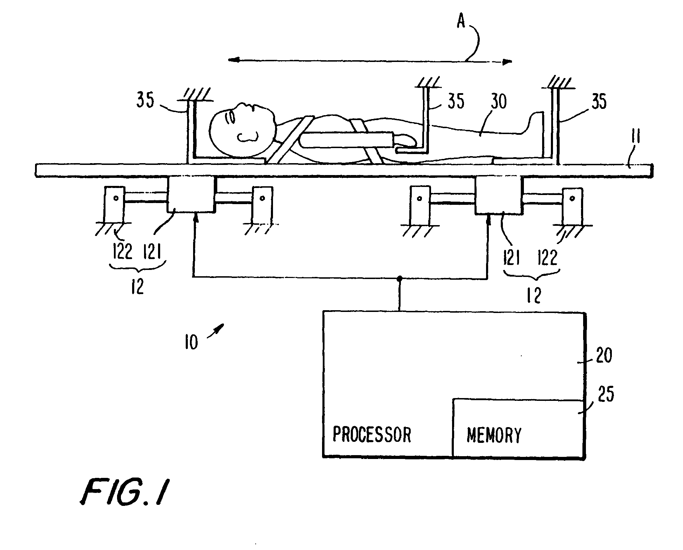

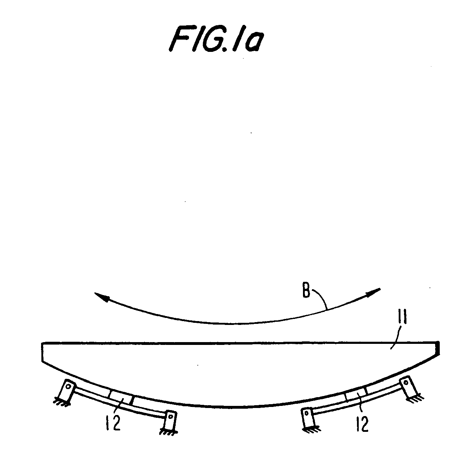

Referring initially to Fig. 1, a platform assembly 10 of the present invention for oscillating a subject in a headwards and footwards direction A -- i.e. parallel to or along the elongation or length of the subject's body -- comprises a platform 11 mounted on displacement modules 12. A fixed portion 122 of each displacement module 12 is fixedly connected to a support frame (not shown in Fig. 1). A movable portion 121 of each displacement module 12 is connected to platform 11. The platform 11 may be constructed of any stiff material such, for example, as plywood, metal, or compressed particle board. The displacement modules 12 may take the form of any device operable to controllably effect the desired oscillatory motion of the platform 11 and may, by way of example, be implemented by electric or pneumatic solenoids, AC or DC motors with a linear shaft, or hydraulic actuators. The displacement modules 12 may alternatively comprise double acting rodless air cylinders that can be mounted therebeneath, as for example Origa NR50-32-2020/50x6-B-M (Origa Corporation, Glendale Heights, IL 60138-4818) or similar cylinders. The amplitude, frequency, and waveform characteristics of the motion of the rodless air cylinders connected to the platform are controlled in the disclosed form of the invention by 24V solenoid operated piloted pneumatic valves such as Mac 52A-14-BOA-DM-DDFJ-IJB-416Y (Mac Valves, Inc., Wixom, MI 48393-2892) or similar valves. In this particular embodiment, the displacement modules 12 comprise both the rodless air cylinders and the pneumatic valves. The modules 12 are preferably DC motors to take advantage of their fast on/off response times; one such DC motor that may be used in the APS 113 Electro-Seis and its matching power amplifier APS 124 (APS Dynamics, Inc., Carlsbad, CA 92008).

In the Fig. 1 side view of the platform 11, only two displacement modules 12 are shown. In the preferred embodiment, two additional displacement modules 12 are positioned symmetrically on the side of the platform 11 that is visible in Fig. 1 (i.e. at the four corners of the platform). However, instead of four displacement modules 12, the platform assembly 10 may alternatively comprise a single displacement module 12, as long as the platform 11 is sufficiently supported throughout its reciprocal motion. For instance, the platform may be connected to a single displacement module 12 for moving the platform in an oscillatory motion and the platform may additionally be supported by passive sliding supports to stabilize the platform throughout its oscillatory motion.

The embodiment shown in Fig. 1 depicts displacement modules 12 as generating linear oscillatory motion of the platform 11 in the headward and footward directions depicted by double arrow A. However, the displacement modules 12 may also be connected for providing other types of motion, such as along an arc B as depicted in Fig. 1a. Similarly, the motion may also follow a circular, elliptical, or any other shaped path.

The characteristics of the oscillatory movement of the platform 11 in the headward and footward directions, such as the amplitude, frequency, and acceleration of motion, are controlled by a processor 20 which transmits control signals to the displacement modules 12 to produce a specific platform motion. The processor 20 may comprise a microprocessor system, or an off-the-shelf waveform generator, which interfaces with the available displacement modules 12, such as DC motors. The processor 20 may optionally include means for altering the characteristics of the motion and may include a memory 25 including motion templates for various types of motions; means for altering the motion characteristics, and the memory, are described in further detail below.

A subject 30, such as an infant or an adult, may be placed directly on the platform 11 as shown in Fig. 1. Alternatively, a mattress or other type of cushioning material (not shown in the drawings) may be placed on the platform 11 between the upper surface of the platform 11 and the subject 30, so that the subject 30 lies in a more comfortable environment. Optionally, the platform assembly 10 may be integrated with a conventional crib or bed. The subject 30 may be held onto the platform 11 with a harness 18. In an alternate embodiment, the head, legs and arms of the subject 30 are removed from direct contact with the platform 11 and are supported by supports 35 that lift them away from platform 11. The supports 35 may comprise slings or pedestals that are adjacent to but separated from the platform 11 to support the subject's head, neck, arms, and legs. In this way, only the torso of the subject 30 is directly affected by the motion of the platform 11.

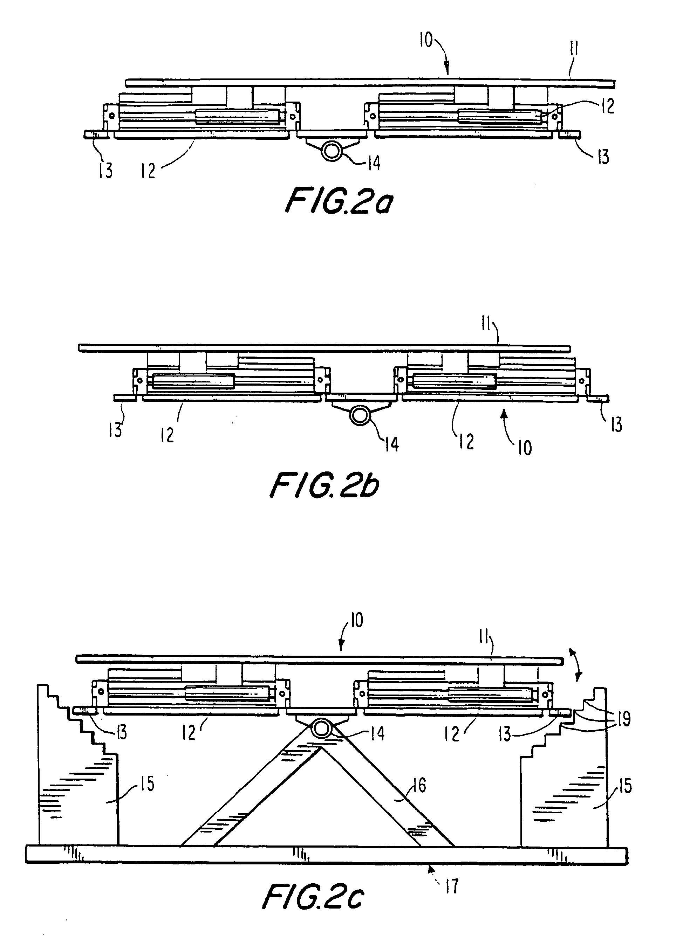

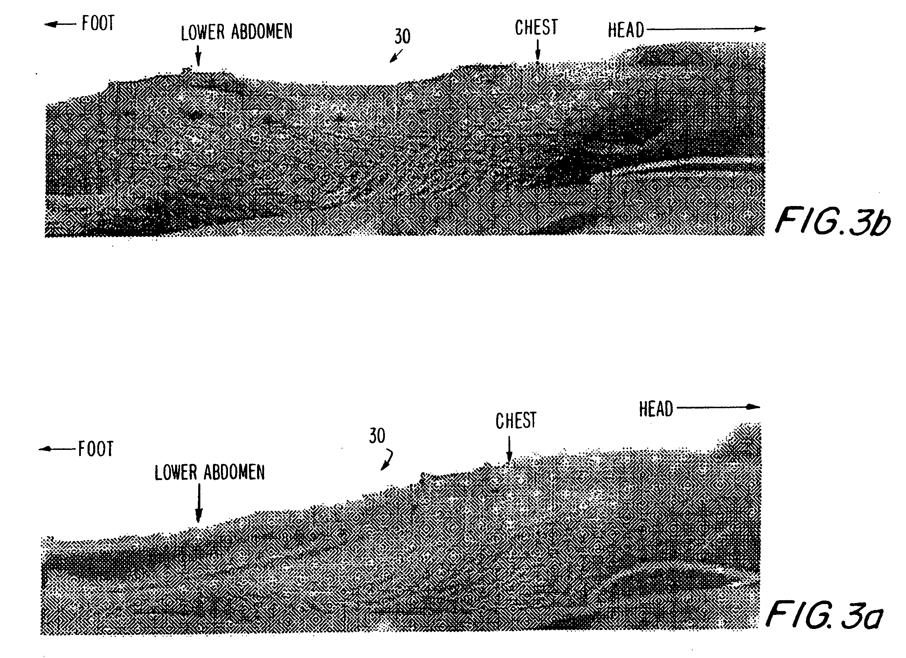

Figures 2a and 2b are more detailed views of the platform 11 and displacement modules 12. Fig. 2a shows the maximum displacement of platform 11 to the right with respect to the displacement modules 12, and Fig. 2b shows the maximum displacement of the platform 11 to the left. Corresponding Figures 3a and 3b respectively depict a side view of a supported subject 30 at the maximum displacement positions of the platform 11. The motion of the platform creates a paradoxical movement of the subject's abdomen and chest; that is, one direction of the motion causes the abdomen to move sufficiently outward and the chest to move sufficiently inward, while the opposite direction of motion causes the opposite effect.

Referring now to Fig. 2c, the platform assembly 10 includes side mounts 13 and a central mount 14 for fixedly connecting the displacement modules 12 to a support frame 17. In Fig. 2c, the frame 17 is shown as having a pair of stepped supports 15 on which the side mounts 13 rest and a fulcrum support 16 to which the central mount 14 is pivotally connected. Each of the stepped supports 15 includes a plurality of steps 19 to which the side mounts 13 are supportably connectable. The position of stepped supports 15 is adjustable so that the platform 11 is selectively tiltable with respect to the frame 17 by +/-18° and can be fixed in a tilted orientation. Thus, the subject 30 on platform 11 may have his or her head, or feet, elevated relative to the other. Fig. 2c shows the platform 11 in its central substantially horizontal position.

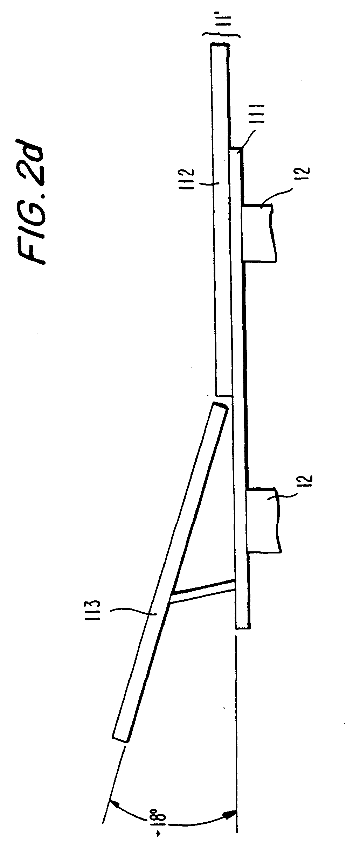

Instead of tilting the entire platform 11, portions of platform 11 may be inclined so that only the subject's upper body, or feet, are inclined. The embodiment shown in Fig. 2d includes a platform 11' having a support portion 111 and two separately inclinable platform sections 112, 113 each individually inclinable, for example, by +18°. Fig. 2d illustratively depicts platform section 113 in the fully inclined position. Although sections 112 and 113 are shown as inclinable upward, they may also be inclined downward from their horizontal position.

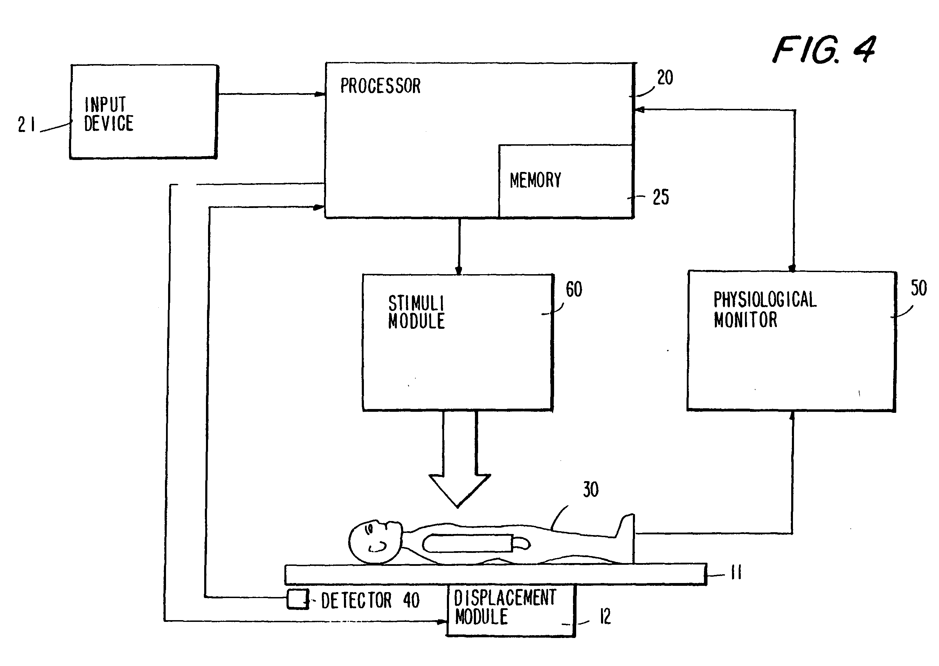

Referring now to the block diagram of Fig. 4, the processor 20 is signally connected to the displacement module 12, which operatively reciprocates or oscillates the platform 11 as described above. The processor 20 may optionally be connected to an input device 21 such as a keyboard, continuously variable dials, or button presets for altering the frequency, amplitude, acceleration, and/or shape of the waveform of the oscillatory motion. Input device 21 may be embodied in a separate unit from the processor 20 or as an integral part thereof. An optional detector 40 may be utilized as a control device for providing feedback to the processor for correcting its output signals to produce the desired movement of the platform 11. The detector 40 and processor 20 may also be employed as a safety device in which the controller stops the motion of the platform if the detector detects that a characteristic of the motion has exceeded a predetermined limit. Detector 40 may be implemented as an accelerometer, a speedometer, a position detector, or any other type of device that detects the particular characteristics of the motion of platform 11. Although the detector is shown as monitoring the movement of platform 11, it may also be connected for monitoring movement of the subject 30.

In another embodiment, the processor 20 includes or is connected to associated memory 25 that stores one or more templates for producing different types of motions. In this embodiment, the user selects one of the stored templates using the input device 21 and may further adjust the characteristics of the template using the input device 21 to alter the frequency, amplitude, and/or acceleration of platform 11. The memory device 25 may be read only memory or read/write memory in which the user can store his or her own motion templates.

One of the motions of which the platform assembly 10 is capable is a reciprocal motion in the headward and footward directions of the subject 30 at approximately .3 - 1.5 Hz for inducing sleep. Another motion is a mixed frequency motion of .3 - 1.5 Hz; this mixed frequency motion is preferably effected while a subject is asleep on the platform assembly so as to minimize the occurrence of apneas.

Also shown in Fig. 4 is a physiologic monitor 50 for monitoring physiological characteristics of the subject 30. The physiologic monitor 50 and processor 20 may be used to detect adverse cardiorespiratory events, such as central, mixed, and obstructive apneas, prolonged apnea, pulse waveform validated arterial oxygen saturation, heart rate changes from an electrocardiogram, severe hypoxemia, and/or severe bradycardia. Processor 20 is programmed to respond to the detection of an adverse event by rapidly shaking the platform 11 in a non-uniform motion from 2-4 Hz to wake the subject 30 and end the adverse event.

If the adverse event is not terminated by the shaking motion, additional stimulus modules 60 may be activated serially or in parallel with the rapid shaking of the platform 11, depending on the severity of the adverse event. The additional stimulus modules 60 may include visual stimulation such as a strong light source directed at the face of the subject, aural stimulation such as a voice sound calling to the subject to wake up, and a physical stimulation such as a pulsating air column directed onto the skin. Other types of stimuli directly contacting the subject may also be used; however, the preferred method for terminating adverse events is to employ stimuli that do not directly contact the subject because of potential trauma which can be caused by direct contact with the body.

The monitoring device 50 is preferably based on Respitrace® technology and includes, but is not limited to, plethysmography, electrocardiography, and pulse wave validated pulse oximetry. Instead of Respitrace® technology, monitor 50 may take the form of any type of device for monitoring the physiological signals that are indicative of the physiological processes of the subject 30, such as mercury in silastic strain gauges and inductive circumferential transducers.

If the subject is an infant, the monitoring device 50 may also detect a crying infant with the processor 20 automatically responding by initiating a rocking motion of the platform 11 to soothe the baby at low, sinusoidal like frequencies from about 0.3 - 1.5 Hz.

Since motion imparted to the subject's torso by the reciprocating platform 11 will appear as deflections in various physiological waveforms, e.g., respiration, electrocardiogram, electroencephalogram, electromyogram, blood pressure, central venous pressure, thoracocardiograph, etc., it is necessary to filter these deflections from the detected waveforms to allow examination of the primary physiological waveform being monitored if this is of interest to the operator. Filtering may be accomplished by adaptive noise cancellation. A cancellation signal is derived from the detector 40 that records at least one of linear displacement, velocity, and acceleration of the movements of platform 11. An adaptive algorithm in the processor 20 then generates a reference noise signal that comprises an estimate of the noise in the primary signal received at the input of the adaptive filter and subtracts the reference noise signal from the subject-originated signal to provide an accurate estimation of the physiological signal.

Movement of platform 11 may also be triggered from the heartbeat of the subject 30 to provide motions within desired portions of the cardiac cycle or from respiration of the subject 30 to provide motions within desired portions of the respiratory cycle. When platform 11 is to be triggered using the heartbeat as the primary signal, monitor 50 may comprise an electrocardiogram or blood pressure monitor. For respiratory system triggering, monitor 50 may comprise an external single or dual band Respitrace® system positioned over the torso or a pneumotachograph air flow sensor located at the airway and mounted on one end of a bias air or oxygen flow system. Feedback loops from the primary physiological signal to the processor 20 implement such triggering controls.

The platform 11 is operable for vibrating at or near the resonance frequency of the subject's respiratory system, such as in the range of about 3 - 12 Hz, to provide assisted ventilation and/or as a sole support of ventilation. The assertion that the platform assembly 10 can provide sole support of ventilation is based upon experiments carried out in anesthetized piglets whose respiratory muscles were paralyzed with intravenous pancuronium.

For the anesthetized piglet experiments, a platform 11 was attached to a displacement module 12 comprising a linear displacement D.C. motor for reciprocally moving the subject 30 to and fro in a horizontal, headward-footward direction, to achieve ventilation without connection to the airways, which is referred to as non-invasive motion ventilation (NIMV) by Nims of Miami Beach, Florida. With significant amplitude of platform movement (i.e. 1.5-2.5 cm) and frequencies of 3-12 Hz, the abdomen of subject 30 moves outward and the ribcage moves inward during one phase of the stroke, and vice versa in the opposite direction of the stroke (see Figs. 3a & 3b). The experiments utilized anesthetized, paralyzed, normal piglets 3-5 days old and 1.5-2 kg in weight.

A tracheotomy was performed for measuring volume and providing PEEP of 5 cm H₂O . To control for changes in PaO₂, which might occur as a result of NIMV, a bias flow of 100% FiO₂ was maintained. Baseline values of arterial blood gases were obtained using conventional mechanical ventilation at frequency of 10 breaths per minute, and mean airway pressure of 4.3cm H₂0. After paralysis with pancuronium bromide, the piglets were started on NIMV at frequencies of 4 to 10 HZ. The frequency of reciprocation remained constant during the subsequent 2 hrs. The following table shows the measurements made during the experiments (*p< 0.05 Bl vs. Time) showing the mean X and the standard deviation (SD) for each measurement:

| | **B1** | **5min** | **15min** | **30min** | **60min** | **90min** | **120min** |
|---|---|---|---|---|---|---|---|
| **pH*** | 7 . 2 2 | 7 . 3 7 | 7.44 4 | 7 . 4 8 | 7 . 4 7 | 7 . 3 7 | 7 . 3 8 |
| | (14) | (.11) | (.09) | (.11) | (.17) | (.11) | (0.12) |
| **PaCO**_{**2**}***** | 5 5 . 7 | 3 1 . 8 | 2 7 . 9 | 2 5 . 2 | 2 8 . 1 | 3 1 . 9 | 3 3 . 2 |
| | (13) | (5.2)* | (6.7)* | (7.2)* | (12.8)* | (15.2)* | (17.1)* |
| **PaO**_{**2**}***** | 2 8 4 | 3 8 2 | 3 7 5 | 3 7 8 | 359 (69) | 3 0 8 | 3 2 3 |
| | (122) | (70) | (69) | (64) | | (115) | (134) |
| **Mean Blood Press mmHg** | 1 1 7 | 1 1 6 | 1 1 6 | 1 1 7 | 109 (50) | 109 (46) | 117 (48) |
| | (46) | (45) | (48) | (48) | | | |
| **Heart Rate beats/min** | 1 5 1 | 1 6 0 | 1 6 4 | 1 4 6 | 140 (28) | 144 (75) | 130 (9) |
| | (45) | (52) | (59) | (27) | | | |

| | | | | | | | |
|---|---|---|---|---|---|---|---|
| * pH=arterial blood pH, Pa CO₂= arterial pH, PaCO₂= arterial carbon dioxide tension, PaO₂= arterial oxygen tension | | | | | | | |

A peak to trough airway pressure measured at the proximal end of the tracheotomy ranged from 1.5 to 3.6 cm H₂0 during NIMV. The stable normal values of arterial blood gases exhibited during NIMV indicate that the method maintains ventilation without the risk of barotrauma from positive pressure applied to the airway.

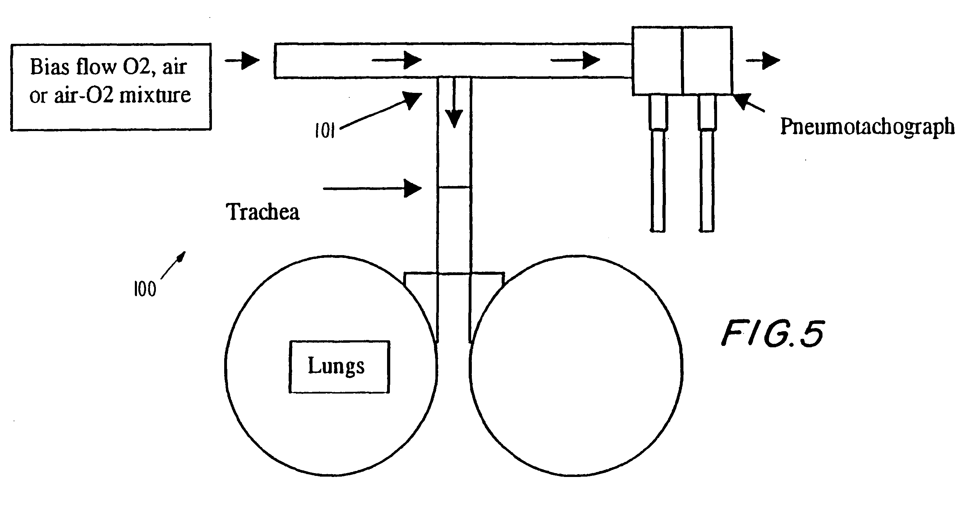

The volume per breath caused by each stroke of the platform 11 was determined by integrating the pneumotachograph airflow waveform obtained at the bias flow circuit 100 attached to the airway through a T-piece 101 as shown in Fig. 5. In the preferred embodiment, Continuous Positive Airway Pressure (CPAP) is provided by the bias flow circuit 100 in conjunction with NIMV to stabilize alveoli openings and promote carbon dioxide removal to the atmosphere from the airway opening.

In piglets, the tidal volume (volume of air per breath) at the airway was found to be approximately 75 to 50% of the predicted tidal volume for piglets by weight with the lower values at the higher frequencies of movement (10Hz) and the higher values at lower frequencies of movement (3Hz). The amplitude of platform movement was greater with lower frequencies than higher frequencies.

The tidal volumes generated by NIMV using the reciprocating platform 11 are lower than the spontaneous tidal volumes at higher respiratory frequencies than the subject normally breathes. However, these tidal volumes generated by the reciprocating platform 11 enabled adequate ventilation, as confirmed by the measurement of normal values of arterial blood gases, i.e. PaCO2 and PaO2. Furthermore, the pressure required to inflate the lungs using the reciprocating platform 11 is less that for conventional positive pressure mechanical ventilators or positive pressure high frequency oscillatory ventilators. The decrease in required pressure attendant with the reciprocating platform 11 minimizes lung damage that may occur as a consequence of positive pressure inflations with mechanical ventilators, a phenomenon designated "barotrauma" and "volutrauma." Therefore, the anesthetized piglet experiment indicates that the reciprocating platform 11 is an effective ventilatory supportive device.

A further experiment was performed to test the effectiveness of NIMV in the presence of abnormal, stiffened lungs. This was evidenced by satisfactory ventilatory support in three anesthetized, paralyzed piglets in which a suspension of 20% human meconium was instilled into the tracheas. The model for this meconium instilled piglet experiment has its basis in the Meconium Aspiration Syndrome (MAS) which is a common cause of respiratory distress among newborn infants. Approximately 5% of the more than 500,000 meconium-stained babies born in the United States each year develop MAS. One third of these infants develop respiratory failure requiring mechanical ventilation. Neonates with MAS requiring mechanical ventilation have mortality rates of up to 60% and death is often related to a persistent pulmonary hypertension of the newborn that is commonly associated with MAS. Meconium is thought to inactivate endogenous surfactant, the surface active agent lining the pulmonary alveoli.

In the meconium instilled experiment, the three piglets that received intratracheal instillation of meconium exhibited the expected physiological responses, as noted in the medical literature when this agent is administered to anesthetized animals. There was an immediate reduction in respiratory compliance (change of volume divided by change of pressure) signifying increased stiffness of the lungs. Profound hypoxemia also occurred. Cardiac output as reflected from arterial minus mixed venous oxygen content differences decreased. The reciprocating platform 11 using NIMV almost restored PaO2 back to normal levels in one animal at 30 minutes following meconium instillation and at 2 hours in the other two despite failure to increase cardiac output. This phenomenon may relate to a regeneration of surfactant and/or a decrease in pulmonary arterial hypertension caused by the meconium by high-frequency NIMV. The decrease in pulmonary arterial hypertension resulted from increased shear stress on the endothelium due to NIMV induced pulsation in blood vessels leading to increaed nitric oxide release. This improves ventilation to perfusion ratios in the lung with consequent improved arterial oxygenation. In terms of surfactant deficiency, it has been found with several different types of high frequency, positive pressure ventilators that there is significantly less severe histologic alterations than with conventional mechanical ventilation. The decreased severity of atelectasis (collapse of lung tissue), which might be associated with surfactant inactivation, was most striking when compared to animals that were on conventional mechanical ventilators. It may be that high frequency, positive pressure ventilators produced internal vibrations, similar to chest physiotherapy, that mitigated the obstructive nature of meconium. There was also a large volume of normal appearing lung tissue in the animals that received meconium despite not suctioning the tracheobronchial tree to remove it. Finally, the time course of restoration of arterial oxygenation with the reciprocating platform using NIMV was much earlier in time than the timing from reports in the literature using high frequency, positive pressure ventilators.

In the anesthetized piglet model, and in normal adults, non-invasive monitoring with a commercial respiratory inductive plethysmography system (Respitrace°, Nims, Miami Beach, FL) using rib cage and abdominal transducers to obtain frequency and volume of oscillations is unsatisfactory for two reasons. The first is the low sampling rate (50 points per second) that does not permit adequate resolution of respiratory signals as high as 10Hz or greater. This problem is obviated with a respiratory inductive plethysmograph sampling at rates of 200 points/sec. The second problem is the nature of the torso oscillations that are produced by the reciprocating platform 11.

Paradoxical movements of the rib cage and abdominal compartments of the respiratory system occur as the platform 11 reciprocates causing multiple degrees of freedom of motion of the respiratory system. This breathing pattern invalidates calibrating the Respitrace® respiratory inductive plethysmograph during natural breathing or during conventional mechanical ventilation at a normal rate and requires that the calibration be performed during high frequency NIMV. With the respiratory inductive plethysmograph sampling at rates of 200 points/sec connected to a single transducer covering the entire torso, this problem also is obviated. Instead of a respiratory inductive plethysmograph, monitor 50 may comprise other technologies which utilize transducers that can be fabricated to cover the entire torso such as mercury in silastic strain gauges and inductance circumferential transducers.

In addition to high frequency NIMV, the platform 11 may also be utilized for ventilatory support at frequencies near normal breathing rates, which in adults is 12-22 breaths per minute and in newborns is 30-60 breaths per minute. In this application, the reciprocating platform 11 may be used as a sole ventilatory support or for ventilatory assistance to spontaneous breaths in subjects who are breathing with inadequate ventilation. In an embodiment for ventilatory assistance, the movement of platform 11 is triggered when an inspiratory threshold is crossed as measured by monitor 50 from a respiratory waveform or its derivative that originates from the respiratory inductive plethysmograph or a pneumotachograph located at the airway. The augmentation may also be set to trigger on a set time of apnea, at a fixed rate or on a selected fraction of spontaneous breaths, as for example every second breath, every fifth breath, or other intervals or periods.

Platform 11 may also be used at higher frequencies in a vibratory NIMV mode. In this further mode of operation, the reciprocating platform 11 cycles at about 15-40Hz with strokes of small amplitude, approximately 2-4 mm in length or more and is used for chest wall vibration. Chest wall vibration has two main applications: 1) use in conjunction with tracheal gas insufflation (via a catheter placed within the trachea to deliver oxygen or an air-oxygen mixture), and 2) to alleviate dyspnea (a subject's unpleasant awareness of shortness of breath) in lung disease. The vibratory tidal volumes are in the range of 0.1-0.2 ml per Kg of body weight. For comparison, the high frequency NIMV mode (4-10Hz) of reciprocating platform 11 described above provides tidal volumes to piglets on the order of 3-5 ml per Kg of body weight. In the vibratory NIMV mode, reciprocating platform 11 provides the same small tidal volumes as the known vibratory plate or disc in any body posture. The reciprocating platform 11 has a maximum vibration frequency of about 40 Hz but distributes the vibratory sensation over the entire torso, a potential advantage over known physiotherapy vibrating discs which are only 25 mm in diameter.

In the first mentioned application of the vibratory NIMV mode, the vibrations aid in diffusion of the gasses in the lungs. In the second application, vibration of the chest wall inspiratory muscles during inspiration (in-phase) reduces breathlessness associated with hypercapnia and resistive loading in normal subjects and patients with chronic obstructive lung disease. Vibration reduces breathlessness because it produces increased neural afferent information from the respiratory system. Application of the vibratory sensation over the entire torso with the platform 11 will greatly influence the afferent information. The vibration during inspiration can be triggered from a monitor 50 such as the Respitrace® plethysmorgraph or pneumotochographic breath waveforms.

The platform 11 may also be fitted with a stationary bicycle so that exercise can be carried out while vibratory NIMV is applied. This arrangement allows patients with chronic lung diseases, such as chronic obstructive lung disease and pulmonary fibrosis, to maintain their physical fitness.

NIMV induced by the reciprocating platform 11 also produces non-ventilatory effects to the lungs. These effects include 1) an increased synthesis in the alveoli and release of surfactant into the airways from the alveoli, 2) an even distribution of pharmacological agents within the lungs, and 3) a clearance of retained bronchopulmonary secretions by means of oscillatory two-phase gas-liquid interaction.

Surfactant, phosphatidylcholine, is a surface active agent synthesized and secreted by alveolar type II cells and constitutes an important component of the alveolar lining fluid. Its classic role is to decrease surface tension in alveolar air spaces to a degree that stabilizes opening of alveolar units, the sites at which gas exchange takes place. Without surfactant, the alveoli would collapse leading to a condition termed atelectasis. In premature infants, deficiency of surfactant occurs because of inadequate synthesis; this leads to infant respiratory distress syndrome, a condition marked by massive pulmonary atelectasis, hyaline membranes within the alveoli, and inflammatory changes. Marked hypoxemia occurs and such patients require mechanical ventilatory support. Treatment consists of prenatal maternal administration of dexamethasone if labor commences during 26-32 weeks after gestation in order to enhance endogenous surfactant synthesis in the fetus. At birth, exogenous surfactant is instilled into the lungs of an infant with this condition. This therapy reduces but does not completely eliminate infant mortality; such patients still often require mechanical ventilation and may develop debilitating or fatal intracranial hemorrhages while on mechanical ventilator therapy. Meconium Aspiration Syndrome, a condition in the newborn that inactivates surfactant, may lead to respiratory distress, hypoxemia and death.

Surfactant is found not only in the alveoli but in bronchioles and small airways. In addition to its role in surface-tension reduction in alveoli, surfactant has several other properties. Any condition in babies or adults that is characterized by mucus abnormality, mucociliary transport deficiency, airways obstruction, or bronchoalveolar collapse could benefit from surfactant therapy; examples of these conditions include, among others, bronchial asthma, pulmonary emphysema, cystic fibrosis, and chronic bronchitis. Further, surfactant has anti-inflammatory and bactericidal properties that provide benefits in a variety of lung diseases.

Ventilation enhances movement of surfactant into the airways and hyperpnea (excessive ventilation) increases the activity of cholinephosphate cytidyltransferase, the rate-limiting enzyme in surfactant synthesis. High-frequency NIMV (4-12 Hz) induced with the reciprocating platform 11 has the capability to promote surfactant synthesis and its release from the alveoli into the airways. It can therefore be applied for the treatment of lung diseases marked by surfactant abnormalities as mentioned above.

The even distribution of pharmacological agents within the lungs is also promoted with high frequency NIMV induced with the inventive reciprocating platform 11. This phenomenon is analogous to suspending solid particles in a liquid vehicle by shaking the container. This method could be used in conjunction with medications delivered to the lungs as aerosols, or instilled as liquids, emulsions, or suspensions. Such medications may include among others corticosteroids, antibiotics, bronchodilators, enzymes, antitrypsin, heparin, surfactant, and hormones.

Finally, clearance of bronchopulmonary secretions in the respiratory system is accomplished using high frequency reciprocating motion of the platform 11 by an oscillatory two-phase gas-liquid interaction in the lungs. This is analogous to chest physiotherapy in patients with cystic fibrosis, chronic bronchitis and pneumonia in which the cough is used to propel secretions up the airways. By prolonging the inspiratory phase and shortening the expiratory phase of oscillations, sufficiently high flow rates are developed during expiration to create conditions for transport of secretions by two-phase gas-liquid interaction. The tilting capability of the platform assembly 10 further aids in clearing secretions with the aid of gravity.

In addition to ventilatory effects, the reciprocating platform 11 serves as a device for cardiopulmonary or cardiac support when operated at high respiratory frequencies or normal cardiac frequencies (3-12 Hz). The types of cardiopulmonary or cardiac support include 1) cardiopulmonary resuscitation, 2) counterpulsation, 3) augmentation of cardiac stroke volume, and 4) non-invasive cardiopulmonary bypass. This mode of operation is called Non Invasive Motion (NIM) Cardiopulmonary or Cardiac Support.

With respect to cardiopulmonary resuscitation, operation of the reciprocating platform 11 in the high frequency NIMV mode during a cardiac arrest of subject 30 distorts the chest wall of the subject and thereby actively compresses and decompresses the heart. During a stroke of the reciprocating platform 11 in one direction the chest wall moves outward paradoxically to the abdomen; during a stroke in the opposite direction the chest moves inward paradoxically to the abdomen.

In the experiments discussed above, NIMV supported ventilation in anesthetized paralyzed piglets for at least 2 hours. The platform 11 used in those experiments was also used to achieve CardioPulmonary Resuscitation (NIMCPR). The following describes the results of the NIMCR experiments.

In these experiments CPR was performed in two tracheotomized, anesthetized, and paralyzed piglets at the end of an experimental protocol using NIMCPR. Two piglets, 1.8 and 2 kg in weight, were placed on a bias flow of 100% FiO₂, and PEEP + 5cmH₂O. The animals received a lethal dose of KCL, and EKG and blood pressure (BP) measurements confirmed cardiac standstill. The animals remained on NIMCPR at 5 and 10 HZ respectively. NIMCPR was continued for 30 minutes. During that time, the blood gases, pulse pressure difference and mean blood pressure were obtained (see table below). Baseline (BL) measurements on conventional mechanical ventilation (PIP=14, PEEP+2) were done prior to NIMCPR.

| **Time** | pH | | PaCO2 | | PaO2 | | Pulse Press. Difference | | Mean Blood Pressure | |
|---|---|---|---|---|---|---|---|---|---|---|
| **Piglet** | 1 | 2 | 1 | 2 | 1 | 2 | 1 | 2 | 1 | 2 |
| **BL** | 7.43 | 7.19 | 15.2 | 47.3 | 332 | 345 | 24 | 28 | 88 | 102 |
| **10m** | 7.51 | 7.51 | 8.4 | 10 | 182 | 108 | 12 | 14.3 | 18.5 | 9 |
| **20m** | 7.39 | 7.34 | 12.5 | 19.9 | 124 | 46.5 | 11 | 14 | 18.5 | 9 |
| **30m** | 7.31 | 7.08 | 15.7 | 43.1 | 77 | 45 | 7 | 13.9 | 18.5 | 9 |

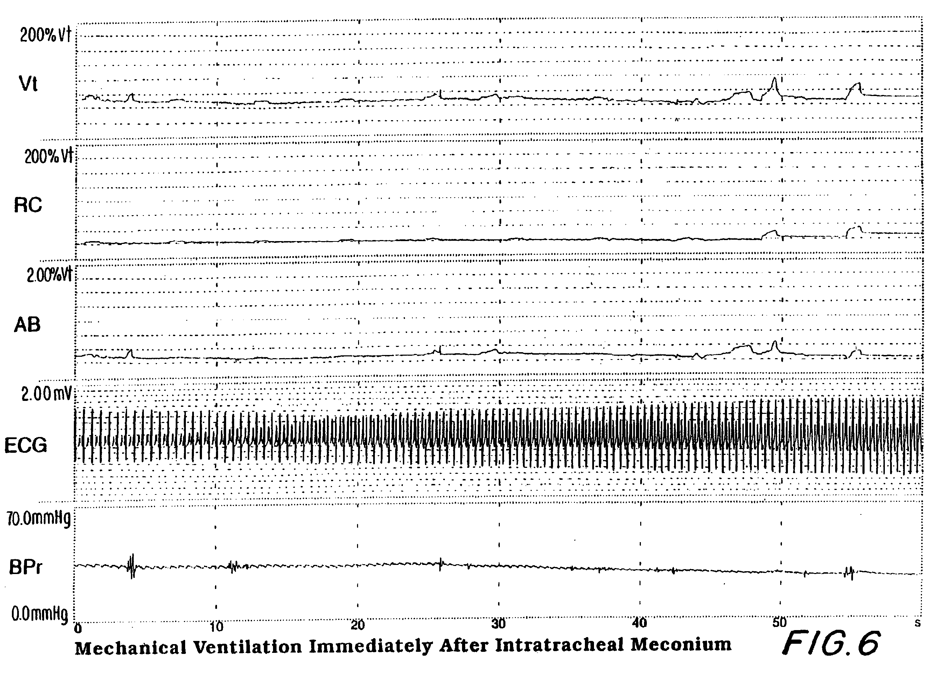

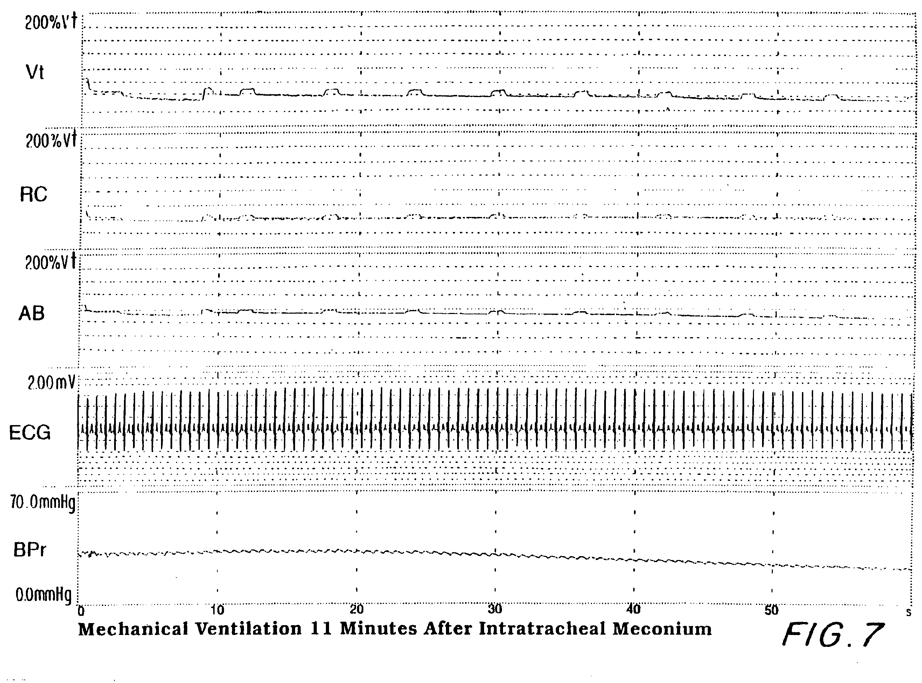

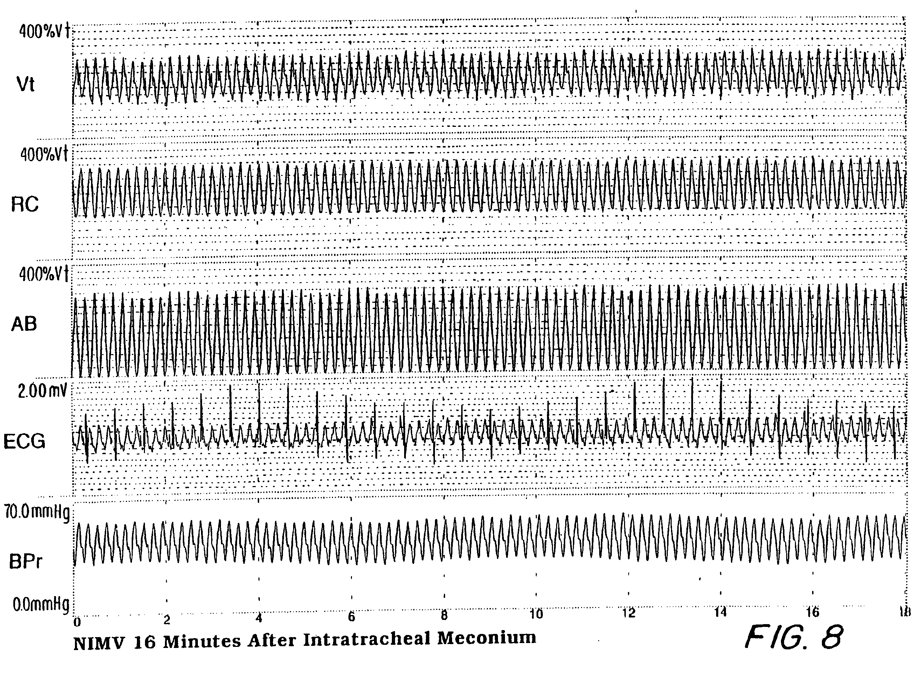

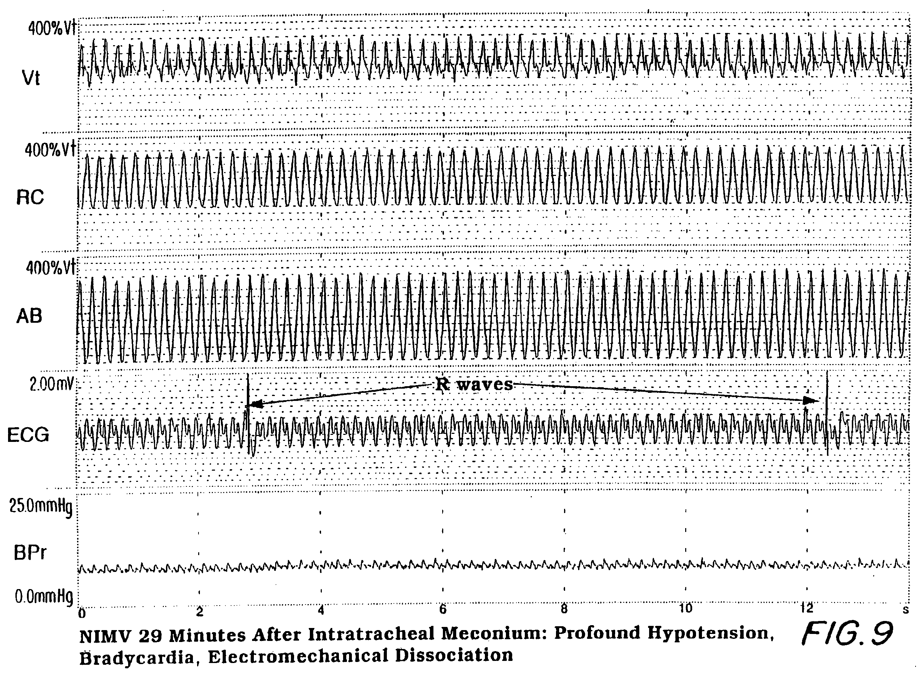

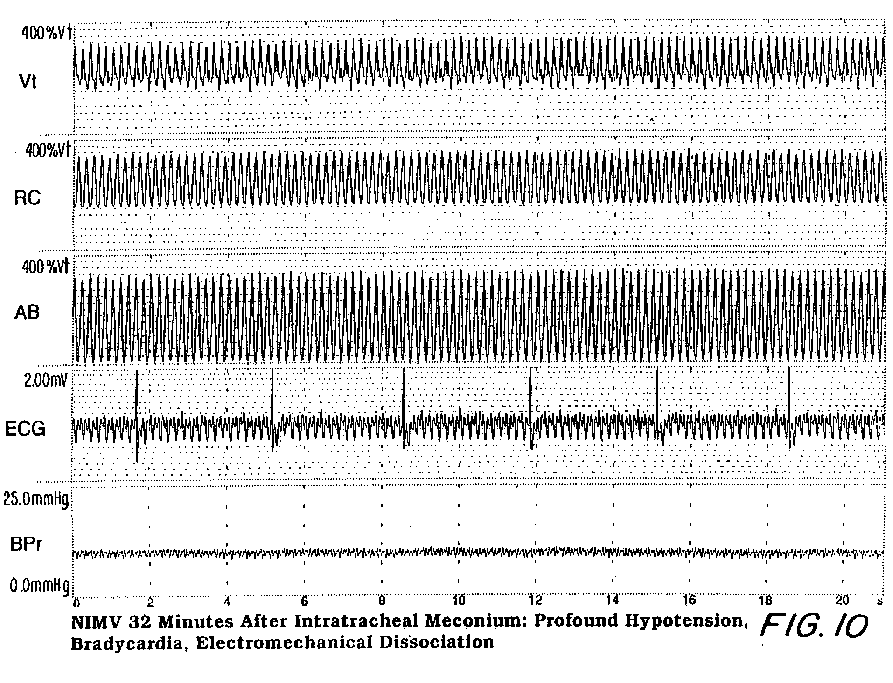

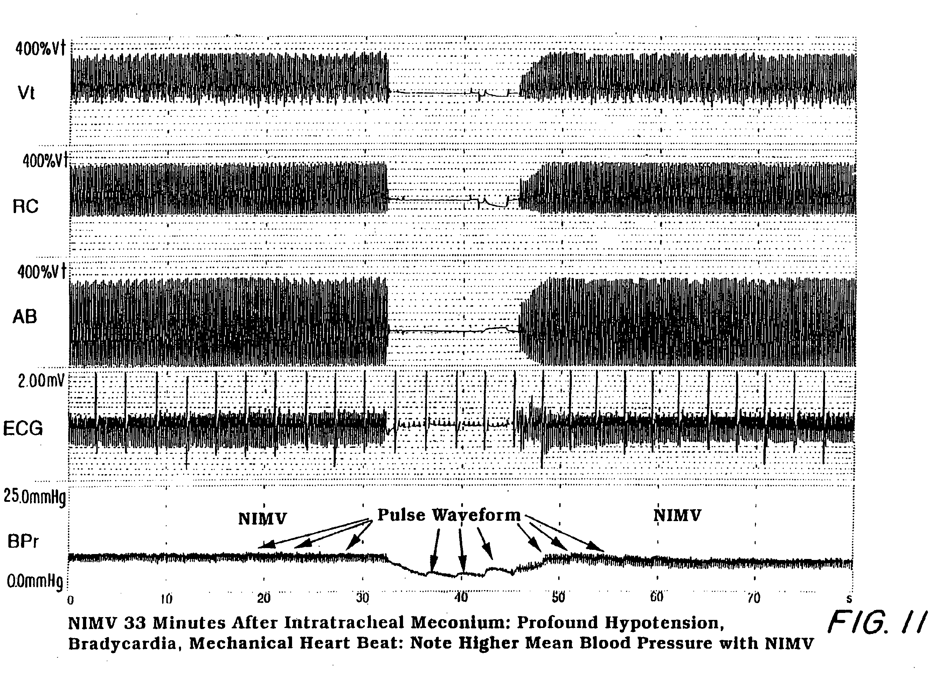

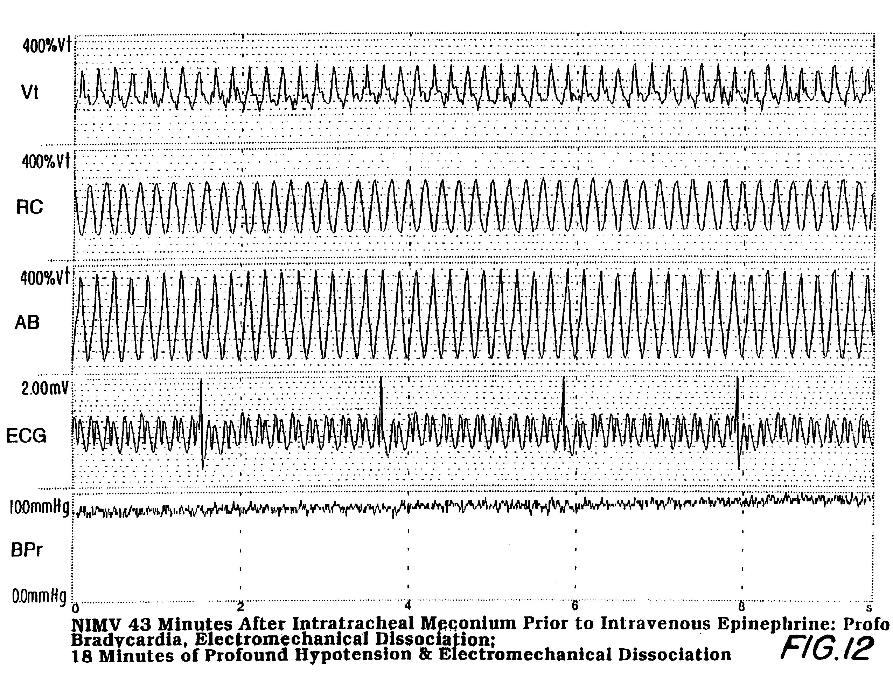

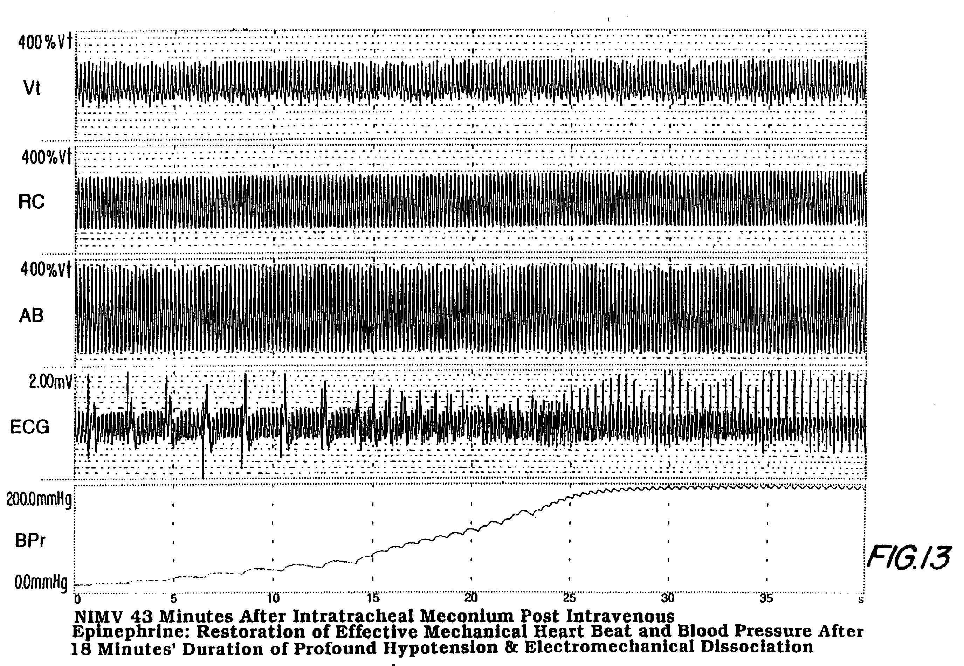

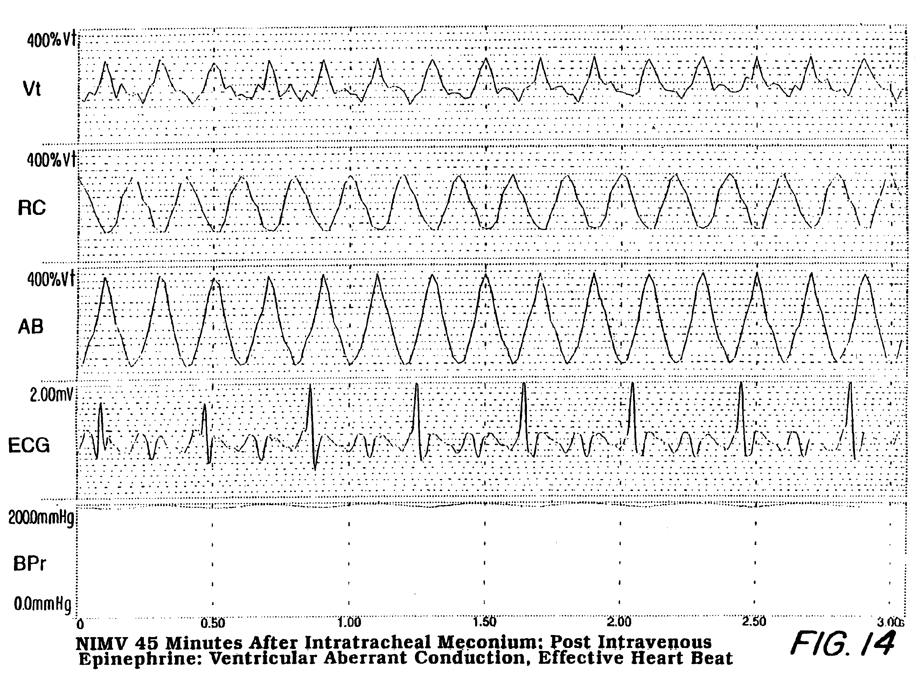

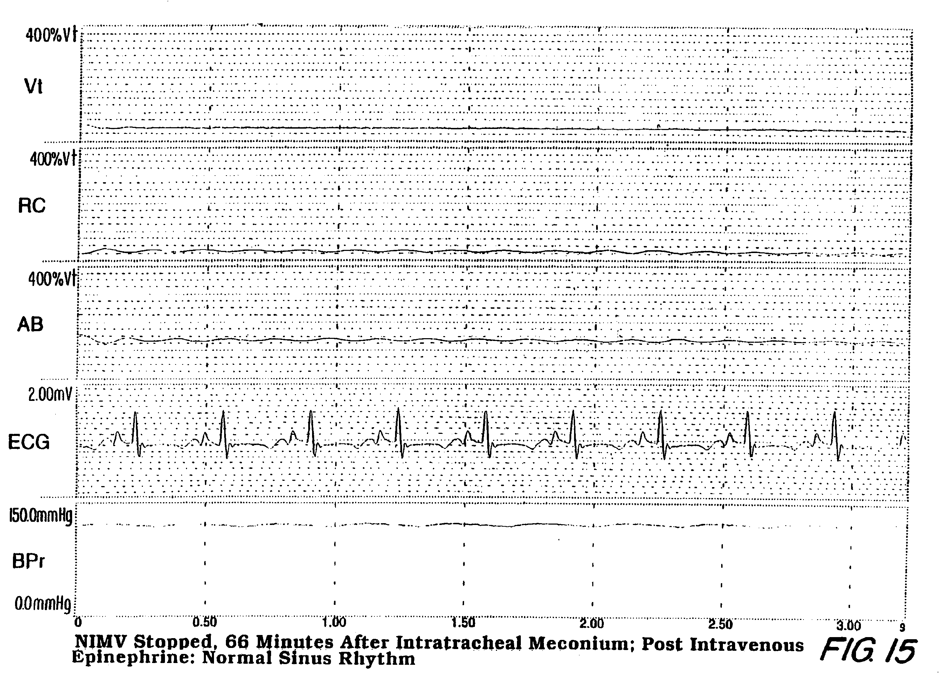

A third animal on NIMV had electromechanical dissociation 10 minutes after intratracheal administration of meconium solution. While NIMV was continued using platform 11, electromechanical dissociation persisted for 18 minutes. (See Figs. 6 through 20 for recordings of the Tidal volume waveform of Respitrace® (Vt), Rib Cage waveform of Respitrace® (RC), Abdominal waveform of Respitrace® (AB), Electrocardiogram (ECG), and Blood Pressure Waveform (BPr) for the third animal). Figs. 6 and 7 show a slowing of the heartbeat (ECG) and a fall in the blood pressure (BPr) after meconium instillation. Figs. 8 and 9 show that NIMS produces rapid oscillations on the respiratory plethysmograph waveforms (Vt, RC, and AB) that are not calibrated to volume because of the multiple degrees of freedom of motion of the respiratory attendant with NIMV. Motion artifacts are also present on the ECG waveform. The heartbeat in Fig. 8 is 10 beats/min, whereas it is less than 0.1 beats/min in Fig. 9. Fig. 10 continues to show profound hypotension and electromechanical dissociation. Fig. 11 shows the higher mean blood pressure with NIMV than without it. Pulsations on the blood pressure waveform are also present. Figs. 12 and 13 show restoration of an effective mechanical heartbeat and blood pressure after intravenous administration of epinephrine. The favorable action exerted by the epinephrine indicates that blood must have been circulating from the venous to the arterial sides of the circulation, which demonstrates the presence of an effective forward circulation with NIMV. Fig. 13 shows that the mean blood pressure increased from 9 to 249 mmHg for 3 minutes followed by a gradual decline to 160 mmHg for 20 minutes. Fig. 14 shows that NIMCPR produced a phase shift between the chest (RC) and the abdomen (AB) of approximately 180°. Finally, Fig. 15 shows restoration of the electrocardiogram to normal sinus rhythm with a heart rate of approximately 200 beats/min. Thus, these experiments indicate NIMCPR to be a promising method for achieving noninvasive cardiopulmonary resuscitation.

This new method for CPR is effective because the heart valves are opened during cardiac arrest and the blood flows through the heart in a forward direction owing to closure of venous valves at the superior thoracic inlets. The measured mean blood pressure of the piglets was low during NIMCPR but not out of proportion to an adult considering the newborn status of the piglets. Mean blood pressure in adults is about 100 mm Hg and, during closed chest compression, is 22-28 mm Hg. The normal mean blood pressure of the piglet was about 60 mm Hg and during CPR ranged between 9 and 18 mmHg.

The reciprocating platform 11 may operate as a stand-alone system or the processor 20 may be triggered from a monitor 50 such as an electrocardiogram or other pulse monitor to begin operation should cardiac arrest or ventricular fibrillation take place.

External enhanced counterpulsation is another type of cardiac related medical procedure which can be accomplished using the reciprocating platform 11. Similar effects to those observed with conventional counterpulsation are obtained with the reciprocating platform. Here, the monitor 50 comprises an electrocardiogram that is used as a trigger, with the platform 11 moving in a direction such that the chest moves outward to effect increased blood flow during diastole and in the opposite direction during systole to cause systolic unloading. This method of delivering a pulse is designated Non-Invasive Motion Pulse (NIMP).

The effects of yet another procedure, high frequency jet ventilation, may also be obtained with the reciprocating platform 11 triggered by the electrocardiogram to augment cardiac stroke volume using NIMP.

Based upon successful application of NIMCPR for CPR, the reciprocating platform 11 can also be used as a means for non invasive cardiopulmonary bypass to operate on a non-beating heart, in conjunction with minimally invasive cardiac surgery. Since the motion of platform 11 can be set to a regular periodic motion, robotic control of the operating instruments synchronized to the platform motion will enable a still heart through principles of virtual reality. That is, the heart and the operating instruments would not move relative to each other.

Operation of the reciprocating platform 11 also causes endothelial stress which releases beneficial mediators in the vascular system. The release of these beneficial mediators normally occurs during physical exercise. Therefore, the NIMV using the reciprocating platform 11 may substitute for the beneficial effects of exercise for those who can not exercise because of physical constraints or respiratory difficulties. Prostaglandins, which are released as a result of increased endothelial stress, play a role in the male erection function; thus, utilization of the platform 11 in this manner may also aid impotency.

By cycling various frequencies of the platform 11 in patients who sleep on the system, relief from the restless leg syndrome and painful legs and moving toes syndrome is possible because the movements of the platform simulate limb activity, which is known to relieve these symptoms.

Finally, according to an NIH publication provided by the National Digestive Diseases Information Clearing House on Constipation, lack of exercise can lead to constipation. For example, the publication states that constipation often occurs after an accident or during an illness when one is bedridden and cannot exercise. The reciprocating platform 11 can be operated to shake the abdomen in a manner analogous to or simulative of the up and down movement that takes place with walking and running and, therefore, may similarly promote bowel movements.

Thus, while there have shown and described and pointed out fundamental novel features of the invention as applied to a preferred embodiment thereof, it will be understood that various omissions and substitutions and changes in the form and details of the devices illustrated, and in their operation, may be made by those skilled in the art without departing from the spirit of the invention. For example, it is expressly intended that all combinations of those elements and/or method steps which perform substantially the same function in substantially the same way to achieve the same results are within the scope of the invention. Moreover, it should be recognized that structures and/or elements and/or method steps shown and/or described in connection with any disclosed form or embodiment of the invention may be incorporated in any other disclosed or described or suggested form or embodiment as a general matter of design choice. It is the intention, therefore, to be limited only as indicated by the scope of the claims appended hereto.

## Claims

1. A platform assembly for moving an animal subject in an oscillatory motion, comprising:
a frame; and
a displacement module having a stationary part and a movable part operatively movable relative to said stationary part, said stationary part being fixedly connected to said frame;
a platform for receiving the subject supportably on the platform and connected to said movable part for selected movement of the platform In an oscillatory motion with operative movement of said movable part;
**characterized in that** It further comprises a controller operatively connected to said displacement module for controlling at least the frequency and the amplitude of the movement of the movable part to selectively induce an oscillatory motion of the platform in a substantially headwards-footwards direction to provide at least one of ventilatory assistance to the subject, ventilatory support of the subject, cardiopulmonary/cardiac support of, and/or assistance to, the subject including cardiopulmonary resuscitation and non-invasive cardiopulmonary bypass, and increased endothelial shear stress for releasing beneficial mediators In a vascular system of the subject.

2. The platform assembly of claim 1, wherein said frequency and amplitude of oscillatory motion of the platform are selected for inducing sleep in the subject.

3. The platform assembly of claim 1, wherein said frequency and amplitude of oscillatory motion of the platform are selected for one of minimizing and preventing occurrence of apneas in the subject.

4. The platform assembly of claim 1, further comprising a physiological monitor for monitoring a physiological signal of the subject and signally connected to said controller, and said controller being operable for controlling the movement of said platform In response to the physiological signal received by the physiological monitor.

5. The platform assembly of claim 4, wherein said physiological monitor Is operable for monitoring the subject to detect an adverse event and the controlled movement of the movable part effects on the platform a shaking motion for waking the subject In response to detection of the adverse event,

6. The platform assembly of claim 5, wherein the adverse event comprises one of central, mixed, and obstructive apneas, pulse waveform validated arterial oxygen saturation, heart rate changes, severe hypoxemia", and severe bradycardla.

7. The platform assembly of claim 5, further comprising means for activating an additional stimulatory module in response to detection of the adverse events wherein said additional stimulatory module comprises one of a strong light source directed at a face of the subject, an auditory stimulation, and a pulsating air column directed on the skin of the subject.

8. The platform assembly of claim 4, wherein said physiological monitor comprises an electrocardiogram and said controlled movement is responsive to the electrocardiogram for providing counterpulsation to the subject.

9. The platform assembly of claim 4, wherein said physiological monitor comprises an electrocardiogram and said controlled movement is responsive to the electrocardiogram for augmenting a cardiac stroke volume of the subject.

10. The platform assembly of claim 1, wherein said controlled movement is selected for eliciting In the subject non-ventilatory effects comprising one of increasing synthesis of alveoli of the subject, evenly distributing pharmacological agents In a respiratory system of the subject, and clearing retained secretions In the respiratory system of a subject.

11. The platform assembly of claim 1, wherein said frequency and amplitude of oscillatory motion of the platform are selected for treating restless leg syndrome and painful legs and moving toes syndrome of the subject.

12. The platform assembly of claim 1, wherein said controlled movement effects a sinusoidal movement of the platform to cause an outward movement of the subject's abdomen along with an Inward movement of the subject's chest in one direction of platform movement and opposite effects on the subject in opposite direction of platform movement for one of assisting and supporting ventilation of the subject.

13. The platform assembly of claim 12, further comprising a continuous positive pressure breathing apparatus with a bias air or oxygen flow operatively connectable to said subject.

14. The platform assembly of claim 1, wherein said controlled movement effects on the platform a vibration with a frequency In the range of 30-40 Hz for achieving ventilation of the subject with tracheal gas diffusion.

15. The platform assembly of claim 1, wherein said controlled movement effects on the platform vibrations with a frequency in the range of 30-40 Hz for reducing perception of breathlessness of the subject for a subject having a lung disease.

16. The platform assembly of claim 1, wherein said controlled movement effects a movement of the platform that causes outward movement of an abdomen of the subject and Inward movement of a chest of the subject in one direction of platform movement and an opposite effect in opposite direction of platform movement for cardiopulmonary resuscitation of the subject when a heart of the subject has arrested or experienced ventricular fibrillation.

17. The platform assembly of claim 16, further comprising a continuous positive pressure breathing apparatus with a bias air or oxygen flow operatively connectable to said subject.

18. The platform assembly of claim 1, wherein said controlled movement effects on the platform a movement which effects paradoxical movement of an abdomen and chest of the subject for Increasing motility of intestines of the subject.

19. The platform assembly of claim 4, wherein said physiological monitor comprises one of a device for use of respiratory inductive plethysmography, electrocardiography, and pulse wave validated pulse oximetry.

20. The platform assembly of claim 4, wherein said physiological monitor operatively monitors at least one physiologic waveform from the group of waveforms comprising respiration, electrocardiogram, electroencephalogram, electromyogram, blood pressure, central venous pressure, and thoracocardiograph.

21. The platform assembly of claim 1, wherein said displacement module comprises one of an electric solenoid, a pneumatic solenoid, an electric motor with a linear shaft, and a hydraulic actuator.

22. The platform assembly of claim 1, further comprising a detector for detecting one of a position, speed, and acceleration of the platform and transmitting an output signal to said controller.

23. The platform assembly of claim 22, further comprising means for filtering deflections In the physiological waveforms of the subject caused by the motion of the platform assembly.

24. The platform assembly of claim 23, wherein said means for filtering deflections comprises an adaptive noise cancellation filter receiving the output signal transmitted from said detector..

25. The platform assembly of claim 1, further comprising an input device operatively connected to said controller for adjusting one of the frequency, amplitude and acceleration of the motion of said platform.

26. The platform assembly of claim 25, further comprising a memory connected to said controller and storing a plurality of templates for Inducing selected controlled movements of said movable part for effecting different types of predetermined motion of the platform, wherein the input device is operable for selecting among the plural templates.

27. The platform assembly of claim 1, wherein the platform Is tiltable relative to the frame so as to elevate one of the feet and head of the subject relative to the other.

28. The platform assembly of claim 1, wherein the platform comprises a first part and a second part; and wherein said first part is tiltable relative to the second part for elevating one of the feet and head of the subject relative to the other.

29. The platform assembly of claim 1, wherein said oscillatory motion comprises one of a linear motion, an arcuate motion, a circular motion, and an elliptical motion.

30. The platform assembly of claim 1,
**characterized in that** it further comprises a monitor operatively connected for monitoring a physiological characteristic of the subject and outputting a monitor signal In response to the physiological characteristic, said controller receiving said monitor signal to selectively Induce an oscillatory motion of the platform In response to said monitor signal In a substantially headwards-footwards direction with respect to the subject,
thereby providing at least one of ventilatory assistance to the subject, ventilatory support of the subject, cardiopulmonary/cardiac support of, and/or assistance to, the subject Including cardiopulmonary resuscitation and non-invasive cardiopulmonary bypass, and increased endothelial shear stress for releasing beneficial mediators In a vascular system of the subject.

31. The oscillating platform assembly of claim 30, wherein said moving means is operable for moving the platform through an oscillatory motion having a predetermined frequency and speed selected for one of assisting ventilation of the subject, supporting ventilation of the subject, and providing cardiopulmonary/cardiac support for the subject.

32. The oscillating platform assembly of claim 31, wherein the cardlopulmonary/cariac support comprises one of cardiopulmonary resuscitation and non-Invasive cardiopulmonary bypass, and Increased endothelial stress in the vascular system of the subject.

33. The oscillating platform assembly of claim 30, wherein said oscillatory motion comprises one of a high-frequency oscillatory motion, a low-frequency oscillatory motion, and a vibratory motion.

34. A method for providing support or assistance to one of a cardioresplrabory function and a cardiovascular function of a living subject, comprising the steps of:
positioning and holding the living subject on a movable platform so that at least a torso of the living subject is movable in response to a movement of the movable platform; and
Initiating a reciprocating motion of the movable platform;
**characterized in that** it further comprises the step of controlling at least the frequency and the amplitude of the reciprocating motion of the movable platform substantially along a headward and footward direction with respect to the living subject with a controller such that the reciprocating motion provides at least one of ventilatory assistance to the subject, ventilatory support of the subject, cardiopulmonary/cardiac support of, and/or assistance to, the subject including cardiopulmonary resuscitation and non-invasive cardiopulmonary bypass, and Increased endothelial shear stress for releasing beneficial mediators in a vascular system of the subject.

35. The method of claim 34, wherein said step of controlling further comprises controlling said characteristic of the reciprocating motion to Induce a paradoxical movement of a chest and an abdomen of the subject in response to the reciprocating motion.

36. The method of claim 34, wherein said step of controlling a characteristic comprises manually adjusting the controller.

37. The method of claim 36, further comprising the step of recording the characteristics of the manually adjusted reciprocating motion to a memory In the controller.

38. The method of claim 34, wherein said step of controlling a characteristic comprises detecting the characteristic of the motion of the platform using a detector, transmitting a signal representing the detected motion to the controller, and controlling the platform in response to the detected signal.

39. The method of claim 38, wherein said step of controlling further comprises terminating the reciprocating motion if the signal representing the detected motion exceeds a predetermined threshold.

40. The method of claim 34, further comprising the step of monitoring a physiologic sign of the living subject.

41. The method of claim 40, wherein the step of monitoring a physiologic sign comprises using an adaptive noise cancellation filter to filter deflections in the physiologic sign caused by the reciprocating motion.

42. The method of claim 40, wherein said step of initiating the reciprocating motion further comprises initiating the reciprocating motion when an adverse cardiorespiratory or cardiovascular event is detected during said step of monitoring a physiologic sign.

43. The method of claim 42, wherein said step of controlling a characteristic of the reciprocating motion of the platform further comprises controlling the reciprocating motion to wake the living subject for terminating the detected adverse cardiorespiratory or cardiovascular event.

44. The method of claim 40, wherein said step of controlling a characteristic of the reciprocating motion comprises controlling the reciprocating motion In response to the physiologic sign.

45. The method of claim 34, wherein said step of controlling a characteristic of the reciprocating motion of the platform further comprises controlling the characteristic of the reciprocating motion for assisting or supporting ventilation of the living subject.

46. The method of claim 34, wherein said step of controlling a characteristic of the reciprocating motion of the platform further comprises controlling the characteristic of the reciprocating motion for assisting tracheal gas insufflation In the subject.

47. The method of claim 34, wherein said step of controlling a characteristic of the reciprocating motion of the platform further comprises controlling the characteristic of the reciprocating motion for alleviating dyspnea in the subject

48. The method of claim 34, wherein said step of controlling a characteristic of the reciprocating motion of the platform further comprises controlling the characteristic of the reciprocating motion for administering cardiopulmonary resuscitation of the living subject.

49. The method of claim 34, wherein said step of controlling a characteristic of the reciprocating motion of the platform further comprises controlling the characteristic of the reciprocating motion for providing non-invasive cardiopulmonary bypass.

50. The method of claim 34, further comprising the step of monitoring a heart rhythm of the living subject with an electrocardiogram and wherein said step of controlllng a characteristic of the reciprocating motion of the platform further comprises controlling the characteristic of the reciprocating motion in response to the electrocardiogram for augmenting cardiac stroke volume.

51. The method of claim 34, further comprising the step of monitoring a heart rhythm of the living subject with an electrocardiogram and wherein said step of controlling a characteristic of the reciprocating motion of the platform further comprises controlling the characteristic of the reciprocating motion In response to the electrocardiogram for increasing blood flow In the living subject.

52. The method of claim 34, wherein said step of controlling a characteristic of the reciprocating motion of the platform further comprises
controlling the characteristic of the reciprocating motion for producing endothelial stress.

53. The method of claim 34, wherein said step of controlling a characteristic of the reciprocating motion of the platform further comprises controlling the characteristic of the reciprocating motion for increasing the motillty of intestines of the living subject.

## Patentansprüche

1. Plattformanordnung zum Bewegen einer lebenden Person in einer Schwingbewegung, umfassend:
einen Rahmen; und
eine Verschiebeeinheit einem ortsfesten Teil und einem bewegbaren Teil, das im Betrieb relativ zu dem ortsfesten Teil bewegbar ist, wobei das ortsfeste Teil fest mit dem Rahmen verbunden ist;
eine Plattform, die die Person tragend auf der Plattform aufnimmt und die mit dem bewegbaren Teil verbunden ist, um die Plattform selektiv in einer Schwingbewegung mit wirksamer Bewegung des bewegbaren Teils zu bewegen;
**dadurch gekennzeichnet, daß** sie des weiteren eine Steuereinheit umfaßt, die funktionell mit der Verschiebeeinheit verbunden ist, um zumindest die Frequenz und die Amplitude der Bewegung des bewegbaren Teils zu steuern, um wahlweise eine Schwingbewegung der Plattform in einer im wesentlichen kopfwärts-fußwärts Richtung auszulösen, um mindestens eines von einer belüftungsmäßigen Hilfe für die Person, einer belüftungsmäßigen Unterstützung für die Person, einer kardiopulmonalen/kardialen Unterstützung und/oder Hilfe für die Person einschließlich einer kardiopulmonalen Reanimierung und eines nicht invasiven kardiopulmonalen Bypasses sowie einer erhöhten endothelialen Scherspannung zum Freisetzen günstiger Überträgersubstanzen in einem Gefäßsystem der Person zu leisten.

2. Plattformanordnung nach Anspruch 1, wobei die Frequenz und die Amplitude der Schwingbewegung der Plattform gewählt werden, um die Person zum Schlafen zu bringen.

3. Plattformanordnung nach Anspruch 1, wobei die Frequenz und die Amplitude der Schwingbewegung der Plattform für die Minimierung oder die Verhinderung des Auftretens von Atemstillständen bei der Person gewählt werden.

4. Plattformanordnung nach Anspruch 1, ferner umfassend einen physiologischen Monitor zum Überwachen eines physiologischen Signals der Person, der signalmäßig mit der Steuereinheit verbunden ist, und wobei die Steuereinheit so betrieben werden kann, daß sie die Bewegung der Plattform in Antwort auf das mit dem physiologischen Monitor empfangene physiologische Signal steuert.

5. Plattformanordnung nach Anspruch 4, wobei der physiologische Monitor so betrieben werden kann, daß er die Person überwacht, um ein nachteiliges Ereignis zu erfassen, und wobei die gesteuerte Bewegung des bewegbaren Teils an der Plattform eine Schüttelbewegung zum Aufwecken der Person als Reaktion auf die Erfassung des nachteiligen Ereignisses ausführt.

6. Plattformanordnung nach Anspruch 5, wobei das nachteilige Ereignis eines von einem zentralen, kombinierten und behindernden Atemstillstand, mit Pulswellen validierter arterieller Sauerstoffsättigung, Herzfrequenzänderungen, schwerer Hypoxämie und schwerer Bradykardie umfaßt.

7. Plattformanordnung nach Anspruch 5, ferner umfassend ein Aktivierungsmittel für ein zusätzliches Stimulationsmodul als Reaktion auf die Erfassung des nachteiligen Ereignisses, wobei das zusätzliche Stimulationsmodul eines von einer auf das Gesicht der Person gerichteten starken Lichtquelle, einer Gehörstimulierung und einer auf die Haut der Person gerichteten pulsierenden Luftsäule umfaßt.

8. Plattformanordnung nach Anspruch 4, wobei der physiologische Monitor ein Elektrokardiogramm umfaßt und die gesteuerte Bewegung auf das Elektrokardiogramm ansprechen kann, um eine Gegenpulsation bei der Person zu erzeugen.

9. Plattformanordnung nach Anspruch 4, wobei der physiologische Monitor ein Elektrokardiogramm umfaßt und die gesteuerte Bewegung auf das Elektrokardiogramm ansprechen kann, um das Herzschlagvolumen der Person zu erhöhen.

10. Plattformanordnung nach Anspruch 1, wobei die gesteuerte Bewegung gewählt wird, um bei der Person nicht-belüftungsmäßige Wirkungen auszulösen, die eines von einer zunehmenden Lungenbläschensynthese bei der Person, einer gleichmäßigen Verteilung pharmakologischer Mittel in dem Atmungssystem der Person und der Abführung von in dem Atmungssystem der Person festgehaltenen Sekreten umfassen.

11. Plattformanordnung nach Anspruch 1, wobei die Frequenz und die Amplitude der Schwingbewegung der Plattform gewählt werden, um das "restless-leg"-Syndrom und schmerzende Beine und das Syndrom der sich bewegenden Zehen bei der Person zu behandeln.

12. Plattformanordnung nach Anspruch 1, wobei durch die gesteuerte Bewegung eine sinusförmige Bewegung der Plattform bewirkt wird, um eine auswärtsgerichtete Bewegung des Unterleibs der Person zusammen mit einer inwärtsgerichteten Bewegung des Brustkorbs der Person in einer Bewegungsrichtung der Plattform und entgegengesetzte Wirkungen an der Person in Gegenrichtung zu der Bewegung der Plattform zum Zweck von einer von einer Hilfe und einer Unterstützung für die Belüftung der Person hervorzurufen.

13. Plattformanordnung nach Anspruch 12, des weiteren mit einem Atmungsgerät mit kontinuierlichem Überdruck, bei dem ein gerichteter Luft- oder Sauerstoffstrom funktionell an die Person anschließbar ist.

14. Plattformanordnung nach Anspruch 1, wobei durch die gesteuerte Bewegung an der Plattform eine Vibration mit einer Frequenz im Bereich von 30 - 40 Hz ausgeübt wird, um eine Belüftung der Person mit Gasdiffusion in die Luftröhre zustande zu bringen.

15. Plattformanordnung nach Anspruch 1, wobei durch die gesteuerte Bewegung an der Plattform Vibrationen mit einer Frequenz im Bereich von 30 - 40 Hz bewirkt werden, um bei einer Person mit einer Lungenkrankheit die Empfindung von Atemnot bei der Person zu vermindern.

16. Plattformanordnung nach Anspruch 1, wobei durch die gesteuerte Bewegung eine Bewegung der Plattform bewirkt wird, welche eine Bewegung des Unterleibs der Person nach außen und eine Bewegung des Brustkorbs der Person nach innen in einer Bewegungsrichtung der Plattform und eine entgegengesetzte Wirkung in Gegenrichtung zu der Bewegung der Plattform zwecks kardiopulmonaler Reanimierung der Person hervorzurufen, wenn das Herz der Person stehengeblieben ist oder ein Herzkammerflimmern erlitten hat.

17. Plattformanordnung nach Anspruch 16, des weiteren mit einem Atmungsgerät mit kontinuierlichem Überdruck, bei dem ein gerichteter Luft- oder Sauerstoffstrom funktionell an die Person anschließbar ist.

18. Plattformanordnung nach Anspruch 1, wobei durch die gesteuerte Bewegung an der Plattform eine Bewegung bewirkt wird, durch welche eine paradoxe Bewegung des Unterleibs und der Brust der Person zur Erhöhung der Beweglichkeit des Gedärms der Person hervorgerufen wird.

19. Plattformanordnung nach Anspruch 4, wobei der physiologische Monitor eine von einer Vorrichtung zur Verwendung für die induktive Atmungsplethysmographie, die Elektrokardiographie und die mit Pulswellen validierte Oximetrie umfaßt.

20. Plattformanordnung nach Anspruch 4, wobei der physiologische Monitor funktionell mindestens eine physiologische Wellenform aus der Gruppe von Wellenformen überwacht, welche Atmung, Elektrokardiogramm, Elektroenzephalogramm, Elektromyogramm, Blutdruck, Zentralvenendruck und Thoraxkardiographie umfassen.

21. Plattformanordnung nach Anspruch 1, wobei die Verschiebeeinheit eines von einem elektrischen Solenoid, einem pneumatischen Solenoid, einem Elektromotor mit einer linearen Welle und einem hydraulischen Stellglied umfaßt.

22. Plattformanordnung nach Anspruch 1, des weiteren mit einem Detektor zum Erfassen eines von einer Position, einer Geschwindigkeit und einer Beschleunigung der Plattform und zum Senden eines Ausgangssignals zu der Steuereinheit.

23. Plattformanordnung nach Anspruch 22, des weiteren mit einem Filtermittel für Ablenkungen in den physiologischen Wellenformen der Person, die durch die Bewegung der Plattformanordnung hervorgerufen werden.

24. Plattformanordnung nach Anspruch 23, wobei das Filtermittel für Ablenkungen einen adaptiven Rauschunterdrückungsfilter umfaßt, der das von dem Detektor gesendete Ausgangssignal empfängt.

25. Plattformanordnung nach Anspruch 1, des weiteren mit einer Eingabevorrichtung, die zum Einstellen einer von der Frequenz, der Amplitude und der Beschleunigung der Plattform funktionell mit der Steuereinheit verbunden ist.

26. Plattformanordnung nach Anspruch 25, des weiteren mit einem Speicher, der mit der Steuereinheit verbunden ist und eine Mehrzahl von Vorlagen zum Auslösen ausgewählter gesteuerter Bewegungen des bewegbaren Teils zur Ausführung verschiedener Arten einer vorgegebenen Bewegung der Plattform speichert, wobei die Eingabevorrichtung zum Zweck einer Wahl unter den mehreren Vorlagen betreibbar ist.

27. Plattformanordnung nach Anspruch 1, wobei die Plattform relativ zu dem Rahmen kippbar ist, um eines von den Füßen und dem Kopf der Person relativ zu dem anderen gehoben werden.

28. Plattformanordnung nach Anspruch 1, wobei die Plattform einen ersten Teil und einen zweiten Teil umfaßt; und wobei der erste Teil relativ zu dem zweiten Teil kippbar ist, um eines von den Füßen oder dem Kopf der Person relativ zu dem anderen zu heben.

29. Plattformanordnung nach Anspruch 1, wobei die Schwingbewegung eine von einer linearen Bewegung, einer bogenförmigen Bewegung, einer kreisförmigen Bewegung und einer elliptischen Bewegung umfaßt.

30. Plattformanordnung nach Anspruch 1,
**dadurch gekennzeichnet, daß** sie des weiteren einen Monitor umfaßt, der zum Überwachen einer physiologischen Eigenschaft der Person und zum Ausgeben eines Monitorsignals als Reaktion auf die physiologische Eigenschaft funktionell angeschlossen ist, wobei die Steuereinheit das Monitorsignal empfängt, um als Reaktion auf das Monitorsignal wahlweise eine Schwingbewegung der Plattform in einer im wesentlichen kopfwärts-fußwärts Richtung in Bezug auf die Person auszulösen und
**dadurch** mindestens eines von einer belüftungsmäßigen Hilfe für die Person, einer belüftungsmäßigen Unterstützung für die Person, einer kardiopulmonalen/kardialen Unterstützung und/oder Hilfe für die Person einschließlich einer kardiopulmonalen Reanimierung und eines nicht invasiven kardiopulmonalen Bypasses sowie einer erhöhten endothelialen Scherspannung zum Freisetzen günstiger Überträgersubstanzen in einem Gefäßsystem der Person zu leisten.

31. Schwingende Plattformanordnung nach Anspruch 30, wobei das Bewegungsmittel betätigbar ist, um die Plattform durch eine Schwingbewegung mit einer vorgegebenen Frequenz und Geschwindigkeit zu bewegen, die für eines von einer Hilfe bei der Belüftung der Person, einer Unterstützung für die Belüftung der Person und der Bereitstellung einer kardiopulmonalen/kardialen Unterstützung für die Person ausgewählt wird.

32. Schwingende Plattformanordnung nach Anspruch 31, wobei die kardiopulmonale/kardiale Unterstützung eines von einer kardiopulmonalen Reanimierung und einem nicht invasiven kardiopulmonalen Bypass sowie einer erhöhten endothelialen Scherspannung in dem Gefäßsystem der Person umfaßt.

33. Schwingende Plattformanordnung nach Anspruch 30, wobei die Schwingbewegung eine von einer hochfrequenten Schwingbewegung, einer niederfrequenten Schwingbewegung und einer Vibrationsbewegung umfaßt.

34. Verfahren zum Leisten von Unterstützung oder Hilfe für eine von einer kardiorespiratorischen Funktion und einer kardiovaskulären Funktion bei einer lebenden Person, mit den folgenden Schritten:
Positionieren und Halten der lebenden Person auf einer bewegbaren Plattform, so daß zumindest der Rumpf der lebenden Person als Reaktion auf eine Bewegung der bewegbaren Plattform bewegbar ist; und
Einleiten einer Hin- und Herbewegung der bewegbaren Plattform;
**dadurch gekennzeichnet, daß** es des weiteren den Schritt des Steuerns von mindestens der Frequenz und der Amplitude der Hin- und Herbewegung der bewegbaren Plattform im wesentlichen entlang einer kopfwärts und fußwärts Richtung in Bezug auf die lebende Person mit einer Steuereinheit derart umfaßt, daß durch die Hin- und Herbewegung mindestens eine von einer belüftungsmäßigen Hilfe für die Person, einer belüftungsmäßigen Unterstützung für die Person, einer kardiopulmonalen/kardialen Unterstützung und/oder Hilfe für die Person einschließlich einer kardiopulmonalen Reanimierung und eines nicht invasiven kardiopulmonalen Bypasses sowie einer erhöhten endothelialen Scherspannung zum Freisetzen günstiger Überträgersubstanzen in dem Gefäßsystem der Person geleistet wird.

35. Verfahren nach Anspruch 34, wobei der Schritt des Steuerns des weiteren das Steuern der Eigenschaft der Hin- und Herbewegung umfaßt, um eine paradoxe Bewegung des Unterleibs und der Brust der Person als Reaktion auf die Hin- und Herbewegung auszulösen.

36. Verfahren nach Anspruch 34, wobei der Schritt des Steuerns einer Eigenschaft das manuelle Einstellen der Steuereinheit umfaßt.

37. Verfahren nach Anspruch 36, des weiteren umfassend den Schritt des Aufzeichnens der Eigenschaften der manuell eingestellten Hin- und Herbewegung in einem Speicher in der Steuereinheit.

38. Verfahren nach Anspruch 34, wobei der Schritt des Steuerns einer Eigenschaft das Erfassen der Eigenschaft der Bewegung der Plattform mit Hilfe eines Detektors, des Sendens eines die erfaßte Bewegung darstellenden Signals zu der Steuereinheit und des Steuerns der Plattform als Reaktion auf das erfaßte Signal umfaßt.

39. Verfahren nach Anspruch 38, wobei der Schritt des Steuerns des weiteren das Beenden der Hin- und Herbewegung umfaßt, wenn das die erfaßte Bewegung darstellende Signal einen Schwellwert überschreitet.

40. Verfahren nach Anspruch 34, des weiteren mit dem Schritt des Überwachens eines physiologischen Zeichens der lebenden Person.

41. Verfahren nach Anspruch 40, wobei der Schritt des Überwachens eines physiologischen Zeichens die Verwendung eines adaptiven Rauschunterdrückungsfilters zum Filtern von Ablenkungen in dem durch die Hin- und Herbewegung hervorgerufenen physiologischen Zeichen umfaßt.

42. Verfahren nach Anspruch 40, wobei der Schritt des Einleitens der Hin- und Herbewegung des weiteren das Einleiten der Hin- und Herbewegung umfaßt, wenn während des Schritts des Überwachens eines physiologischen Zeichens ein nachteiliges kardiorespiratorisches oder kardiovaskuläres Ereignis erfaßt wird.

43. Verfahren nach Anspruch 42, wobei der Schritt des Steuerns einer Eigenschaft der Hin- und Herbewegung der Plattform des weiteren das Steuern der Hin- und Herbewegung umfaßt, um die lebende Person zum Zwecke der Beendigung des erfaßten nachteiligen kardiorespiratorischen oder kardiovaskulären Ereignisses zu wecken.

44. Verfahren nach Anspruch 40, wobei der Schritt des Steuerns einer Eigenschaft der Hin- und Herbewegung das Steuern der Hin- und Herbewegung als Reaktion auf das physiologische Zeichen umfaßt.

45. Verfahren nach Anspruch 34, wobei der Schritt des Steuerns einer Eigenschaft der Hin- und Herbewegung der Plattform des weiteren das Steuern der Eigenschaft der Hin- und Herbewegung als Hilfe oder Unterstützung beim Belüften der lebenden Person umfaßt.

46. Verfahren nach Anspruch 34, wobei der Schritt des Steuerns einer Eigenschaft der Hin- und Herbewegung der Plattform des weiteren das Steuern der Eigenschaft der Hin- und Herbewegung als Hilfe beim Einblasen von Gas in die Luftröhre der Person umfaßt.

47. Verfahren nach Anspruch 34, wobei der Schritt des Steuerns einer Eigenschaft der Hin- und Herbewegung der Plattform des weiteren das Steuern der Eigenschaft der Hin- und Herbewegung zur Linderung von Atemnot bei der Person umfaßt.

48. Verfahren nach Anspruch 34, wobei der Schritt des Steuerns einer Eigenschaft der Hin- und Herbewegung der Plattform des weiteren das Steuern der Eigenschaft der Hin- und Herbewegung zur Anwendung der kardiopulmonalen Reanimierung der lebenden Person umfaßt.

49. Verfahren nach Anspruch 34, wobei der Schritt des Steuerns einer Eigenschaft der Hin- und Herbewegung der Plattform des weiteren das Steuern der Eigenschaft der Hin- und Herbewegung zur Bereitstellung eines nicht invasiven kardiopulmonalen Bypasses umfaßt.

50. Verfahren nach Anspruch 34, des weiteren mit dem Schritt des Überwachens eines Herzrhythmus der lebenden Person mit einem Elektrokardiogramm, und wobei der Schritt des Steuerns einer Eigenschaft der Hin- und Herbewegung der Plattform des weiteren das Steuern der Eigenschaft der Hin- und Herbewegung als Reaktion auf das Elektrokardiogramm umfaßt, um das Herzschlagvolumen zu erhöhen.

51. Verfahren nach Anspruch 34, des weiteren mit dem Schritt des Überwachens eines Herzrhythmus der lebenden Person mit einem Elektrokardiogramm, und wobei der Schritt des Steuerns einer Eigenschaft der Hin- und Herbewegung der Plattform des weiteren das Steuern der Eigenschaft der Hin- und Herbewegung als Reaktion auf das Elektrokardiogramm umfaßt, um den Blutstrom bei der lebenden Person zu erhöhen.

52. Verfahren nach Anspruch 34, wobei der Schritt des Steuerns einer Eigenschaft der Hin- und Herbewegung der Plattform des weiteren das Steuern der Eigenschaft der Hin- und Herbewegung zur Erzeugung einer endothelialen Scherspannung umfaßt.

53. Verfahren nach Anspruch 34, wobei der Schritt des Steuerns einer Eigenschaft der Hin- und Herbewegung der Plattform des weiteren das Steuern der Eigenschaft der Hin- und Herbewegung zur Erhöhung der Motilität der Gedärme der lebenden Person umfaßt.

## Revendications

1. Assemblage de plate-forme destiné à déplacer un sujet animal selon un mouvement oscillatoire, comprenant :
un châssis ; et
un module de déplacement possédant une partie stationnaire et une partie mobile pouvant se déplacer de façon opérationnelle par rapport à ladite partie stationnaire, ladite partie stationnaire étant fixée au châssis ;
une plate-forme destinée à recevoir le sujet de façon supportable sur la plate-forme et reliée à ladite partie mobile pour un déplacement sélectionné de la plate-forme selon un mouvement oscillatoire avec mouvement opérationnel de ladite partie mobile ;
**caractérisé en ce qu'**il comprend en outre un dispositif de commande relié de façon opérationnelle au dit module de déplacement pour commander au moins la fréquence et l'amplitude du mouvement de la partie mobile pour induire de façon sélective un mouvement oscillatoire de la plate-forme dans une direction sensiblement tête-pieds pour fournir au moins une assistance ventilatoire au sujet, un soutien ventilatoire au sujet, un soutien cardio-pulmonaire/cardiaque du sujet et/ou une assistance au sujet, dont une réanimation cardio-pulmonaire et une dérivation cardio-pulmonaire non invasive et une force de cisaillement endothéliale accrue pour relâcher des médiateurs utiles dans le système vasculaire du sujet.

2. Assemblage de plate-forme selon la revendication 1, dans lequel lesdites fréquences et amplitude du mouvement oscillatoire de la plate-forme sont déterminées de façon à provoquer le sommeil chez le sujet.

3. Assemblage de plate-forme selon la revendication 1, dans lequel lesdites fréquences et amplitude du mouvement oscillatoire de la plate-forme sont choisies pour réduire ou prévenir l'apparition d'apnées chez le sujet.

4. Assemblage de plate-forme selon la revendication 1, comprenant en outre un moniteur physiologique pour surveiller un signal physiologique chez le sujet et relié par signal au dit dispositif de commande, ledit dispositif de commande pouvant fonctionner pour commander le déplacement de ladite plate-forme en réponse au signal physiologique reçu par le moniteur physiologique.

5. Assemblage de plate-forme selon la revendication 4, dans lequel ledit moniteur physiologique est opérationnel pour surveiller le sujet afin de détecter un événement indésirable et le mouvement contrôlé de la partie mobile crée sur la plate-forme un mouvement de secousse pour réveiller le sujet en cas de détection d'un événement indésirable.

6. Assemblage de plate-forme selon la revendication 5, dans lequel l'événement indésirable comprend un des événements suivants, à savoir une apnée centrale, mixte ou obstructive, une saturation artérielle en oxygène validée par une forme d'onde d'impulsion, des modifications de la fréquence cardiaque, une grave hypoxémie et une grave bradycardie.

7. Assemblage de plate-forme selon la revendication 5, comprenant en outre un moyen pour activer un module de stimulation supplémentaire en cas de détection de l'événement indésirable, dans lequel ledit module de stimulation supplémentaire comprend une forte source de lumière dirigée sur une face du sujet, une stimulation auditive et une colonne d'air pulsatoire dirigée vers la peau du sujet.

8. Assemblage de plate-forme selon la revendication 4, dans lequel ledit moniteur physiologique comprend un électrocardiogramme et ledit mouvement commandé est sensible à l'électrocardiogramme pour fournir des contrepulsations au sujet.

9. Assemblage de plate-forme selon la revendication 4, dans lequel ledit moniteur physiologique comprend un électrocardiogramme et ledit mouvement commandé est sensible à l'électrocardiogramme pour augmenter le débit systolique cardiaque du sujet.

10. Assemblage de plate-forme selon la revendication 1, dans lequel ledit mouvement commandé est déterminé pour provoquer chez le sujet des effets non ventilatoires comprenant une synthèse croissante d'alvéole du sujet, une distribution uniforme d'agents pharmacologiques dans le système respiratoire du sujet et le retrait de sécrétions persistantes dans le système respiratoire du sujet.

11. Assemblage de plate-forme selon la revendication 1, dans lequel lesdites fréquences et amplitude du mouvement oscillatoire de la plate-forme sont déterminées pour traiter le syndrome des jambes sans repos et les jambes douloureuses et le syndrome des orteils qui bougent chez le sujet.

12. Assemblage de plate-forme selon la revendication 1, dans lequel ledit mouvement commandé entraîne un mouvement sinusoïdal de la plate-forme provoquant un mouvement vers l'extérieur de l'abdomen du sujet et un mouvement vers l'intérieur de la poitrine du sujet, dans un sens du mouvement de la plate-forme, et des effets opposés sur le sujet dans le sens opposé du mouvement de la plate-forme pour assistance ou soutien de la ventilation du sujet.

13. Assemblage de plate-forme selon la revendication 12, comprenant en outre un appareil respiratoire à pression positive continue avec un débit d'air ou d'oxygène en biais pouvant être relié de façon opérationnelle au dit sujet.

14. Assemblage de plate-forme selon la revendication 1, dans lequel ledit mouvement commandé provoque sur la plate-forme une vibration avec une fréquence comprise dans une plage de 30-40 Hz pour réaliser la ventilation du sujet avec une diffusion de gaz trachéale.

15. Assemblage de plate-forme selon la revendication 1, dans lequel ledit mouvement commandé provoque sur la plate-forme des vibrations avec une fréquence comprise dans une plage de 30-40 Hz pour réduire la perception d'oppression du sujet chez un sujet atteint d'une maladie pulmonaire.

16. Assemblage de plate-forme selon la revendication 1, dans lequel ledit mouvement commandé provoque un mouvement de la plate-forme qui entraîne un mouvement vers l'extérieur de l'abdomen du sujet et un mouvement vers l'intérieur de la poitrine du sujet, pour un sens du mouvement de la plate-forme, et un effet opposé dans le sens opposé du mouvement de la plate-forme pour une réanimation cardio-respiratoire du sujet lorsque le coeur du sujet s'est arrêté ou a subi une fibrillation ventriculaire.

17. Assemblage de plate-forme selon la revendication 16, comprenant en outre un appareil respiratoire à pression positive continue avec un débit d'air ou d'oxygène pouvant être relié de façon opérationnelle au dit sujet.

18. Assemblage de plate-forme selon la revendication 1, dans lequel ledit mouvement contrôlé provoque sur la plate-forme un mouvement qui entraîne un mouvement paradoxal de l'abdomen et de la poitrine du sujet pour augmenter la motilité intestinale du sujet.

19. Assemblage de plate-forme selon la revendication 4, dans lequel ledit moniteur physiologique comprend un dispositif pour utiliser une pléthysmographie par inductance respiratoire, une électrocardiographie et une oxymétrie du pouls validée par onde pulsée.

20. Assemblage de plate-forme selon la revendication 4, dans lequel ledit moniteur physiologique surveille de façon opérationnelle au moins une forme d'onde physiologique issue du groupe de formes d'onde comprenant la respiration, l'électrocardiogramme, l'électroencéphalogramme, l'électromyogramme, la pression artérielle, la pression veineuse centrale et le pneumographe.

21. Assemblage de plate-forme selon la revendication 1, dans lequel ledit module de déplacement comprend un solénoïde électrique, un solénoïde pneumatique, un moteur électrique avec un arbre linéaire ou un actionneur hydraulique.

22. Assemblage de plate-forme selon la revendication 1, comprenant en outre un détecteur pour détecter une position, une vitesse ou une accélération de la plate-forme et transmettre un signal de sortie audit dispositif de commande.

23. Assemblage de plate-forme selon la revendication 22, comprenant en outre un moyen pour filtrer des déflections des formes d'onde physiologiques du sujet provoquées par le mouvement de l'assemblage de plate-forme.

24. Assemblage de plate-forme selon la revendication 23, dans lequel ledit moyen pour filtrer les déflections comprend un filtre d'annulation du bruit adaptatif recevant le signal de sortie transmis par ledit détecteur.

25. Assemblage de plate-forme selon la revendication 1, comprenant en outre un dispositif d'entrée relié de façon opérationnelle audit dispositif de commande pour ajuster la fréquence, l'amplitude ou l'accélération du déplacement de ladite plate-forme.

26. Assemblage de plate-forme selon la revendication 25, comprenant en outre une mémoire connectée audit dispositif de commande et stockant une pluralité de modèles pour induire des mouvements commandés déterminés de ladite partie mobile pour réaliser différents types de mouvement prédéterminés de la plate-forme, le dispositif d'entrée étant opérationnel pour effectuer une sélection parmi la pluralité de modèles.

27. Assemblage de plate-forme selon la revendication 1, dans lequel la plate-forme peut basculer par rapport au châssis pour élever les pieds ou la tête du sujet les uns par rapport à l'autre.

28. Assemblage de plate-forme selon la revendication 1, dans lequel la plate-forme comprend une première et une seconde partie et dans lequel ladite première partie peut basculer par rapport à la seconde partie pour élever les pieds ou la tête du sujet les uns par rapport à l'autre.

29. Assemblage de plate-forme selon la revendication 1, dans lequel ledit mouvement oscillatoire comprend un mouvement linéaire, un mouvement arqué, un mouvement circulaire ou un mouvement elliptique.

30. Assemblage de plate-forme selon la revendication 1, **caractérisé en ce qu'**il comprend en outre un moniteur connecté de façon opérationnelle pour surveiller une caractéristique physiologique du sujet et émettre un signal de surveillance en réponse à la caractéristique physiologique, ledit dispositif de commande recevant ledit signal de surveillance pour induire de façon sélective un mouvement oscillatoire de la plate-forme en réponse au dit signal de surveillance dans un sens sensiblement tête-pieds par rapport au sujet, fournissant ainsi au moins une assistance ventilatoire au sujet, un soutien ventilatoire au sujet, un soutien cardio-respiratoire/cardiaque du sujet et/ou une assistance au sujet, dont une réanimation cardio-pulmonaire et une dérivation cardio-pulmonaire non invasive et une force de cisaillement endothéliale accrue pour relâcher des médiateurs utiles dans le système vasculaire du sujet.

31. Assemblage de plate-forme oscillatoire selon la revendication 30, dans lequel ledit moyen de déplacement peut fonctionner pour déplacer la plate-forme avec un mouvement oscillatoire possédant une fréquence et une vitesse prédéterminées sélectionnées pour assister la ventilation du sujet, soutenir la ventilation du sujet et fournir un soutien cardio-pulmonaire/cardiaque au sujet.

32. Assemblage de plate-forme oscillatoire selon la revendication 31, dans lequel le soutien cardio-pulmonaire/cardiaque comprend la réanimation cardio-pulmonaire et la dérivation cardio-pulmonaire non invasive et un stress endothélial accru dans le système vasculaire du sujet.

33. Assemblage de plate-forme oscillatoire selon la revendication 30, dans lequel ledit mouvement oscillatoire comprend un mouvement oscillatoire haute fréquence, un mouvement oscillatoire basse fréquence ou un mouvement de vibration.

34. Procédé destiné à fournir un soutien ou une assistance à une fonction cardio-respiratoire ou à une fonction cardiovasculaire d'un sujet vivant, comprenant les étapes consistant à :
positionner et maintenir le sujet vivant sur une plate-forme mobile de sorte qu'au moins le torse du sujet vivant est mobile en réponse à un mouvement de la plate-forme mobile ; et
amorcer un mouvement de va-et-vient de la plate-forme mobile ;
**caractérisé en ce qu'**il comprend en outre l'étape consistant à commander au moins la fréquence et l'amplitude du mouvement de va-et-vient de la plate-forme mobile sensiblement dans un sens tête-pieds par rapport au sujet vivant avec un contrôleur de sorte que le mouvement de va-et-vient fournit au moins une assistance ventilatoire au sujet, un soutien ventilatoire du sujet, un soutien cardio-pulmonaire/cardiaque du sujet et/ou une assistance au sujet, dont la réanimation cardio-pulmonaire et la dérivation cardio-pulmonaire non invasive, et la force de cisaillement endothéliale accrue pour relâcher les médiateurs utiles dans le système vasculaire du sujet.

35. Procédé selon la revendication 34, dans lequel ladite étape de commande comprend en outre la commande de ladite caractéristique du mouvement de va-et-vient pour induire un mouvement paradoxal de la poitrine et de l'abdomen du sujet en réponse au mouvement de va-et-vient.

36. Procédé selon la revendication 34, dans lequel ladite étape de commande d'une caractéristique comprend l'étape consistant à ajuster manuellement le dispositif de commande.

37. Procédé selon la revendication 36, comprenant en outre l'étape consistant à enregistrer les caractéristiques du mouvement de va-et-vient ajusté manuellement dans une mémoire dans le dispositif de commande.

38. Procédé selon la revendication 34, dans lequel ladite étape de commande d'une caractéristique comprend la détection de la caractéristique du mouvement de la plate-forme à l'aide d'un détecteur, la transmission d'un signal représentant le mouvement de détection au dispositif de commande et la commande de la plate-forme en réponse au signal détecté.

39. Procédé selon la revendication 38, dans lequel ladite étape de commande à contrôler comprend en outre l'arrêt du mouvement de va-et-vient si le signal représentant le mouvement détecté dépasse un seuil prédéterminé.

40. Procédé selon la revendication 34, comprenant en outre l'étape consistant à surveiller un signe physiologique du sujet vivant.

41. Procédé selon la revendication 40, dans lequel l'étape consistant à surveiller un signe physiologique comprend l'utilisation d'un filtre d'annulation de bruit adaptif pour filtrer les déflections du signe physiologique provoquées par le mouvement de va-et-vient.

42. Procédé selon la revendication 40, dans lequel ladite étape consistant à amorcer le mouvement de va-et-vient comprend l'initiation du mouvement de va-et-vient lorsqu'un événement cardio-respiratoire ou cardio-vasculaire indésirable est détecté pendant ladite étape de surveillance d'un signe physiologique.

43. Procédé selon la revendication 42, dans lequel ladite étape de commande d'caractéristique du mouvement de va-et-vient de la plate-forme comprend en outre la commande du mouvement de va-et-vient pour réveiller le sujet vivant pour mettre fin à l'événement cardio-respiratoire ou cardiovasculaire indésirable.

44. Procédé selon la revendication 40, dans lequel ladite étape de commande d'une caractéristique du mouvement de va-et-vient comprend la commande du mouvement de va-et-vient en réponse au signe physiologique.

45. Procédé selon la revendication 34, dans lequel ladite étape de commande d'une caractéristique du mouvement de va-et-vient de la plate-forme comprend en outre la commande de la caractéristique du mouvement de va-et-vient pour assister ou soutenir la ventilation du sujet vivant.

46. Procédé selon la revendication 34, dans lequel ladite étape de commande d'une caractéristique du mouvement de va-et-vient de la plate-forme comprend en outre la commande de la caractéristique du mouvement de va-et-vient pour assister l'insufflation de gaz dans la trachée du sujet.

47. Procédé selon la revendication 34, dans lequel ladite étape de commande d'une caractéristique du mouvement de va-et-vient de la plate-forme comprend en outre la commande de la caractéristique du mouvement de va-et-vient pour soulager la dyspnée chez le sujet.

48. Procédé selon la revendication 34, dans lequel ladite étape de commande d'une caractéristique du mouvement de va-et-vient de la plate-forme comprend en outre la commande de la caractéristique du mouvement de va-et-vient pour administrer une réanimation cardio-respiratoire au sujet vivant.

49. Procédé selon la revendication 34, dans lequel ladite étape de commande d'une caractéristique du mouvement de va-et-vient de la plate-forme comprend en outre la commande de la caractéristique du mouvement de va-et-vient pour obtenir une dérivation cardio-pulmonaire non invasive.

50. Procédé selon la revendication 34, comprenant en outre l'étape consistant à surveiller le rythme cardiaque du sujet vivant avec un électrocardiogramme et dans lequel ladite étape de commande d'une caractéristique du mouvement de va-et-vient de la plate-forme comprend en outre la commande de la caractéristique du mouvement de va-et-vient en réponse à l'électrocardiogramme pour augmenter le débit systolique cardiaque.

51. Procédé selon la revendication 34, comprenant en outre l'étape consistant à surveiller le rythme cardiaque du sujet vivant avec un électrocardiogramme et dans lequel ladite étape de commande d'une caractéristique du mouvement de va-et-vient de la plate-forme comprend en outre la commande de la caractéristique du mouvement de va-et-vient en réponse à l'électrocardiogramme pour augmenter le débit sanguin chez le sujet vivant.

52. Procédé selon la revendication 34, dans lequel ladite étape de commande d'une caractéristique du mouvement de va-et-vient de la plate-forme comprend en outre la commande de la caractéristique du mouvement de va-et-vient pour produire un stress endothélial.

53. Procédé selon la revendication 34, dans lequel ladite étape de commande d'une caractéristique du mouvement de va-et-vient de la plate-forme comprend en outre la commande de la caractéristique du mouvement de va-et-vient pour augmenter la motilité intestinale du sujet vivant.
